(19) Europäisches Patentamt
European Patent Office
Office européen des brevets

(11) EP 1 534 218 B1

(12) # FASCICULE DE BREVET EUROPEEN

(45) Date de publication et mention de la délivrance du brevet:
**16.12.2009 Bulletin 2009/51**

(21) Numéro de dépôt: **03758215.2**

(22) Date de dépôt: **06.08.2003**

(51) Int Cl.:
***A61K 8/31*** (2006.01) ***A61K 8/37*** (2006.01)
***A61K 8/81*** (2006.01) ***A61K 8/88*** (2006.01)
***A61K 8/92*** (2006.01) ***A61Q 1/10*** (2006.01)

(86) Numéro de dépôt international:
**PCT/FR2003/002479**

(87) Numéro de publication internationale:
**WO 2004/022009 (18.03.2004 Gazette 2004/12)**

(54) **COMPOSITION DE MAQUILLAGE DES FIBRES KERATINIQUES NOTAMMENT DES FIBRES KERATINIQUES COMME LES CILS**

SCHMINKE FÜR KERATINFASER, INSBESONDERE FÜR WIMPER

MAKE-UP COMPOSITION FOR KERATIN FIBRES SUCH AS EYELASHES

(84) Etats contractants désignés:
**AT BE BG CH CY CZ DE DK EE ES FI FR GB GR HU IE IT LI LU MC NL PT RO SE SI SK TR**

(30) Priorité: **06.09.2002 FR 0211104**
**06.09.2002 FR 0211096**
**06.09.2002 FR 0211097**
**30.09.2002 FR 0212097**

(43) Date de publication de la demande:
**01.06.2005 Bulletin 2005/22**

(73) Titulaire: **L'ORÉAL**
**75008 Paris (FR)**

(72) Inventeurs:
- **DE LA POTERIE, Valérie**
  **F-77820 Le Châtelet-en-Brie (FR)**
- **PAYS, Karl**
  **94410 Saint-Maurice (FR)**
- **DAUBIGE, Thérèse**
  **37420 Beaumont en Veron (FR)**
- **STYCZEN, Patrice**
  **F-91190 Gif-sur-Yvette (FR)**
- **BICHON, Yohann**
  **94700 MAISONS-ALFORT (FR)**

(74) Mandataire: **Le Coupanec, Pascale A.M.P.**
**Nony & Associés**
**3, rue de Penthièvre**
**75008 Paris (FR)**

(56) Documents cités:
**EP-A- 0 667 146** **EP-A- 1 029 897**
**EP-A- 1 201 221** **WO-A-00/56280**
**WO-A-00/57844** **WO-A-91/12793**
**FR-A- 2 726 467** **US-A- 5 480 632**
**US-A- 5 783 176**

- **PATENT ABSTRACTS OF JAPAN vol. 1998, no. 10, 31 août 1998 (1998-08-31) & JP 10 139631 A (KOKURIYUUDOU:KK;NIPPON SHIKIZAI KOGYO KENKYUSHO:KK), 26 mai 1998 (1998-05-26)**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 534 218 B1

Printed by Jouve, 75001 PARIS (FR)

## Description

La présente invention se rapporte au maquillage de matières kératiniques notamment de fibres kératiniques, comme les cils, les sourcils et les cheveux, et plus particulièrement au maquillage des cils.

La composition selon l'invention peut se présenter sous la forme d'un mascara, d'un produit pour les sourcils, d'un eye-liner ou d'un produit de maquillage des cheveux. Plus spécialement, l'invention porte sur un mascara. Il peut notamment s'agir d'une composition de maquillage, d'une composition à appliquer sur ou sous un maquillage, dites encore respectivement « top-coat » ou « base-coat », ou bien encore d'une composition de traitement des cils.

D'une manière générale, les compositions de maquillage de fibres kératiniques et notamment de cils sont constituées d'au moins une cire ou d'un mélange de cires dispersé dans une phase liquide. C'est principalement à travers la quantité de cire et des autres ingrédients non volatils, reflétée par la teneur en matière sèche de la composition, que sont ajustées les spécificités d'application recherchées pour les compositions, comme par exemple leur fluidité, leur pouvoir couvrant et/ou leur pouvoir recourbant, ainsi que leur pouvoir épaississant (encore appelé pouvoir chargeant ou maquillant).

Il existe en pratique essentiellement deux types de formulation de mascara, à savoir d'une part, des mascaras aqueux, dits « mascaras crèmes », se présentant sous forme d'émulsion de cires dans l'eau et, d'autre part, des mascaras anhydres ou à faible teneur en eau et/ou solvants hydrosolubles, dits « mascaras waterproof », formulés à l'état de dispersion de cires dans des solvants non aqueux. Il faut toutefois noter que certains mascaras se présentant sous forme d'émulsions de cire dans l'eau sont également qualifiés de « waterproof ». Leur résistance à l'eau résulte essentiellement de la présence de quantité importante de latex dans leur composition. Ils se caractérisent en outre par une faible teneur en matière sèche et sont donc très peu maquillants.

La présente invention concerne plus particulièrement le domaine des compositions de maquillage de fibres kératiniques ne contenant pas d'eau ni de solvant hydrosoluble ou à faible teneur en eau et/ou en solvants hydrosolubles, dits « mascaras waterproof », se présentant sous forme de dispersion de cire(s) dans des solvants non aqueux.

Classiquement, ces compositions possèdent une teneur en matière sèche comprise entre 15 % et 45 % environ en poids par rapport au poids total de la composition.

Le document US 5,480,632 décrit des compositions cosmétiques non aqueuses contenant des quantités d'agent dispersant suffisantes pour permettre des quantités plus élevées de matière sèche cosmétiquement acceptable, colorants, cires, poudres sèches, etc...comparativement à des compositions cosmétiques non aqueuses conventionnelles.

Comme précisé précédemment, cette plage de valeur en matière sèche est généralement peu satisfaisante en terme de rendu de maquillage. Or, si l'on cherche à augmenter cette teneur en matière sèche au-delà de cette valeur, on se heurte rapidement à un problème de défaut de fluidité. La composition de maquillage devient trop épaisse à l'application et ne présente en outre plus la déformabilité nécessaire à son application homogène sur toute la surface des cils. Par ailleurs, une observation au microscope montre que dans ce type de composition, les particules de cires se présentent généralement sous forme d'agrégats.

Le but de la présente invention est précisément de proposer une composition de maquillage ou de soin des matières kératiniques possédant une teneur élevée en matière sèche, permettant notamment l'obtention d'un maquillage plus épaississant que celui obtenu avec les compositions de type « waterproof » traditionnelles, tout en préservant une consistance compatible avec l'utilisation en maquillage envisagée.

La présente invention a donc pour objet une composition cosmétique de maquillage de fibres kératiniques selon la revendication 1.

La présente invention a également pour objet un procédé de préparation d'une composition telle que définie précédemment comprenant au moins une étape de dispersion d'au moins une cire à l'état de particules ayant une dimension allant de 0,5 μm à 30 μm, en particulier allant de 1 à 20 μm, dans au moins un solvant non aqueux, ledit solvant étant à une température inférieure à celle de fusion de ladite cire à l'état de particules.

La présente invention se rapporte en outre à un procédé de maquillage des fibres kératiniques, dans lequel on applique sur lesdites fibres kératiniques, notamment les cils, une composition telle que définie précédemment ou telle que qu'obtenue par l'un des procédés tels que définis précédemment.

Avantageusement, les compositions de l'invention présentent de plus une vitesse de séchage plus élevée que les compositions classiques waterproof, ce qui permet bien entendu de réduire le temps nécessaire pour la mise en oeuvre du procédé de maquillage et le risque de décalquage du maquillage des cils sur la paupière adjacente. Cela permet en outre, le cas échéant, de pouvoir appliquer plusieurs couches de ladite composition en un temps satisfaisant et de renforcer ainsi encore l'effet épaississant du maquillage obtenu avec ces compositions.

## CARACTERISATION DE LA MATIERE SECHE

Au sens de la présente invention, la « teneur en matière sèche », désigne la teneur en matière non volatile.

Cette quantité de matière sèche, communément appelée « extrait sec » ou sous sa forme abrégée ES, des compositions selon l'invention, est mesurée par échauffement de l'échantillon par des rayons infrarouges de 2 μm à 3,5 μm de longueur d'onde. Les substances contenues dans lesdites compositions qui possèdent une pression de vapeur élevée, s'évaporent sous l'effet de ce rayonnement. La mesure de la perte de poids de l'échantillon permet de déterminer « l'extrait sec » de la composition. Ces mesures sont réalisées au moyen d'un dessiccateur à infrarouges commercial « LP16® » de chez METTLER. Cette technique est parfaitement décrite dans la documentation de l'appareil fournie par METTLER.

Le protocole de mesure est le suivant :

On étale environ 1g de la composition sur une coupelle métallique. Celle-ci, après introduction dans le dessiccateur, est soumise à une consigne de température de 120 °C pendant une heure. La masse humide de l'échantillon, correspondant à la masse initiale et la masse sèche de l'échantillon, correspondant à la masse après exposition au rayonnement, sont mesurées au moyen d'une balance de précision.

La teneur en matière sèche est calculée de la manière suivante :

**Extrait Sec = 100 x (masse sèche / masse humide).**

Les valeurs mesurées à l'aide du protocole décrit ci-dessus peuvent différer des valeurs théoriques correspondantes de plus ou moins 1 %.

Les compositions selon l'invention se caractérisent notamment par une teneur en matière sèche supérieure à 45 %, notamment allant de 46 à 80 %, en particulier de 48 à 70 % et plus particulièrement de 50 à 65 % en poids par rapport au poids total de la composition.

## CARACTERISATIONS RHEOLOGIOUES

La composition selon l'invention peut être en outre caractérisée par son comportement viscoélastique, notamment à l'aide de différents paramètres rhéologiques.

De façon générale, un matériau est dit viscoélastique quand, sous l'effet du cisaillement, il possède à la fois les caractéristiques d'un matériau purement élastique, c'est à dire capable de stocker de l'énergie et les caractéristiques d'un matériau purement visqueux, c'est à dire capable de dissiper de l'énergie.

Plus particulièrement, le comportement viscoélastique des compositions conformes à l'invention peut être caractérisé par son module de rigidité G, son élasticité $\delta$ et son seuil d'écoulement $\tau_c$ ; ces paramètres sont notamment définis dans l'ouvrage « Initiation à la rhéologie », G. Couarraze et J.L. Grossiord, 2ème édition, 1991, Edition Lavoisier-Tec 1 Doc.

Ces paramètres sont déterminés par des mesures effectuées à 25 °C ± 0,5 °C à l'aide du rhéomètre à contrainte imposée « Haake RheoStress 600® » de la société THERMORHEO, équipé d'un mobile en acier inoxydable à géométrie plan/plan, le plan ayant un diamètre de 20 mm et un entrefer (distance entre le plan inférieur - appelé plan stator - sur lequel est déposée la composition et le plan supérieur - appelé plan rotor) de 0,3 mm. Les deux plans sont striés pour limiter les phénomènes de glissement aux parois des plans.

Les mesures dynamiques sont réalisées en appliquant une variation harmonique de la contrainte. Dans ces expériences, les amplitudes du cisaillement, de la vitesse de cisaillement et de la contrainte sont faibles de manière à rester dans les limites du domaine viscoélastique linéaire du matériau (conditions permettant d'évaluer les caractéristiques rhéologiques de la composition au repos).

Le domaine linéaire viscoélastique est généralement défini par le fait que la réponse du matériau (i.e. la déformation) est à tout moment directement proportionnelle à la valeur de la force appliquée (i.e. la contrainte). Dans ce domaine, les contraintes appliquées sont faibles et le matériau subit des déformations sans modifier sa structure microscopique. Dans ces conditions, le matériau est étudié « au repos » et de façon non destructive.

La composition est soumise à un cisaillement harmonique selon une contrainte $\tau(t)$ variant de façon sinusoïdale selon une pulsation $\omega$ ($\omega = 2\Pi\nu$, ν étant la fréquence du cisaillement appliqué). La composition ainsi cisaillée subie une contrainte $\tau(t)$ et répond selon une déformation $\gamma(t)$ correspondant à des micro déformations pour lesquelles le module de rigidité varie peu en fonction de la contrainte imposée.

La contrainte $\tau(t)$ et la déformation $\gamma(t)$ sont définies respectivement par les relations suivantes :

$$\tau(t) = \tau_0 \cos(\omega \cdot t) \qquad \gamma(t) = \gamma_0 \cos(\omega \cdot t - \delta)$$

$\tau_0$ étant l'amplitude maximale de la contrainte et $\gamma_0$ étant l'amplitude maximale de la déformation. $\delta$ est l'angle de déphasage entre la contrainte et la déformation.

Les mesures sont effectuées à une fréquence de 1 Hz (v= 1 Hz).

On mesure ainsi l'évolution du module de rigidité G (correspondant au rapport de $\tau_o$ sur $\gamma_o$) et de l'élasticité $\delta$ (correspondant à l'angle de déphasage de la contrainte appliquée par rapport à la déformation mesurée) en fonction de la contrainte $\tau(t)$ appliquée.

On mesure en particulier la déformation de la composition pour la zone de contrainte dans laquelle la variation du module de rigidité G et de l'élasticité $\delta$ est inférieure à 7% (zone des microdéformations) et on détermine ainsi les paramètres dits « plateaux » Gp et $\delta_p$. La contrainte seuil $\tau_c$(correspondant à la force minimale qu'il est nécessaire d'appliquer à la composition pour provoquer son écoulement) est déterminée à partir de la courbe $\delta = f(\tau)$ et correspond à la valeur de $\tau$ pour laquelle $\delta (\tau_c) = 1{,}05\ \delta_p$.

Le comportement viscoélastique des compositions selon l'invention est caractérisé par un module de rigidité plateau Gp inférieur ou égal à 28 000 Pa, plus particulièrement inférieur ou égal à 25 000 Pa, voire à 20 000 Pa.

Les compositions conformes à l'invention peuvent par ailleurs posséder un seuil d'écoulement $\tau_c$ allant de 10 Pa à 200 Pa, et en particulier allant de 20 Pa à 100 Pa.

## MILIEU SOLVANT NON AQUEUX

La composition selon l'invention comprend un milieu solvant non aqueux.

Ce milieu est susceptible de former une phase continue et contient, comme son nom l'indique, au moins un solvant non aqueux qui est un composé volatil, non soluble dans l'eau et liquide à température ambiante et pression atmosphérique.

Par « composé volatil », on entend, au sens de l'invention, tout composé (ou milieu non aqueux) susceptible de s'évaporer au contact de la peau ou de la fibre kératinique en moins d'une heure, à température ambiante et pression atmosphérique. Le composé volatil est un composé cosmétique volatil, liquide à température ambiante, ayant notamment une pression de vapeur non nulle, à température ambiante et pression atmosphérique, notamment ayant une pression de vapeur allant de 0,13 Pa à 40 000 Pa ($10^{-3}$ à 300 mm de Hg),en particulier allant de 1,3 Pa à 13 000 Pa (0,01 à 100 mm de Hg), et plus particulièrement allant de 1,3 Pa à 1300 Pa (0,01 à 10 mm de Hg).

Par opposition, on entend par « composé non volatil », un composé restant sur la peau ou la fibre kératinique à température ambiante et pression atmosphérique, au moins plusieurs heures et ayant notamment une pression de vapeur inférieure à $10^{-3}$ mm de Hg (0,13 Pa).

Les composés volatils sont généralement présents en une proportion majoritaire dans le milieu solvant non aqueux, c'est-à-dire qu'ils représentent plus de 50 % en poids dudit milieu solvant non aqueux. En particulier, ils peuvent représenter au moins 60 %, plus particulièrement au moins 70 % et peuvent aller jusqu'à 100 % en poids par rapport au poids total dudit milieu solvant non aqueux.

La teneur en composé volatil, non soluble dans l'eau et liquide à température ambiante est généralement inférieure à 55 %, et est notamment comprise entre 10 à 54 %, en particulier entre 15 et 52 % et plus particulièrement entre 17,5 et 50 % en poids par rapport au poids total de la composition.

Le composé volatil, non soluble dans l'eau et liquide à température ambiante est en particulier une huile ou un solvant organique cosmétiquement acceptable. Par l'expression « cosmétiquement acceptable », on entend un composé dont l'utilisation est compatible avec une application sur les fibres kératiniques et sur la peau.

Bien entendu, le milieu solvant non aqueux de la composition selon l'invention peut comprendre un mélange de tels composés.

Les huiles volatiles peuvent être des huiles hydrocarbonées, des huiles siliconées et/ou fluorées ou leurs mélanges.

On entend par « huile hydrocarbonée », une huile contenant principalement des atomes d'hydrogène et de carbone et éventuellement des atomes d'oxygène, d'azote, de soufre, de phosphore. Les huiles hydrocarbonées volatiles peuvent être choisies parmi les huiles hydrocarbonées ayant de 8 à 16 atomes de carbones, et notamment les alcanes ramifiés en $C_8$-$C_{16}$ comme les isoalcanes en $C_8$-$C_{16}$ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux « d'Isopars®» ou de « Permetyls® », les esters ramifiés en $C_8$-$C_{16}$, le néopentanoate d'isohexyle, et leurs mélanges. D'autres huiles hydrocarbonées volatiles comme les distillats de pétrole, notamment ceux vendus sous la dénomination « Shell Solt® » par la société SHELL, peuvent aussi être utilisées.

Comme huiles volatiles, on peut aussi utiliser les silicones volatiles, comme par exemple les huiles de silicones linéaires ou cycliques volatiles, notamment celles ayant une viscosité $\leq$ 6 centistokes ($6.10^{-6}$ $m^2/s$), et ayant notamment de 2 à 10 atomes de silicium, ces silicones comportant éventuellement des groupes alkyles ou alkoxy ayant de 1 à 22 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

On peut également utiliser des solvants organiques volatils notamment fluorés tels que le nonafluorométhoxy butane ou le perfluorométhylcyclopentane.

Selon un mode de réalisation particulier des compositions selon l'invention, le composé volatil, non soluble dans l'eau et liquide à température ambiante est choisi parmi les huiles volatiles hydrocarbonées ayant de 8 à 16 atomes de carbone et leurs mélanges.

Le milieu solvant non aqueux peut également comprendre au moins un composé non volatil, non soluble dans l'eau et liquide à température ambiante, notamment au moins une huile non volatile, qui peut être en particulier choisie parmi les huiles hydrocarbonées et/ou siliconées et/ou fluorées non volatiles.

Comme huile hydrocarbonée non volatile, on peut notamment citer :

- les huiles hydrocarbonées d'origine végétale telles que les triglycérides constitués d'esters d'acides gras et de glycérol dont les acides gras peuvent avoir des longueurs de chaînes variées de $C_4$ à $C_{24}$, ces dernières pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; ou encore les triglycérides des acides caprylique/caprique comme ceux vendus par la société STEARINERIES DUBOIS ou ceux vendus sous les dénominations de « Miglyol 810® », « 812® » et « 818® » par la société DYNAMIT NOBEL,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane, et leurs mélanges;
- les esters de synthèse comme les huiles de formule $R_1COOR_2$ dans laquelle $R_1$ représente le reste d'un acide gras linéaire ou ramifié comportant de 1 à 40 atomes de carbone et $R_2$ représente une chaîne hydrocarbonée notamment ramifiée contenant de 1 à 40 atomes de carbone à condition que $R_1 + R_2$ soit ≥ 10, comme par exemple l'huile de Purcellin (octanoate de cétostéaryle), le myristate d'isopropyle, le palmitate d'isopropyle, le benzoate d'alcool en $C_{12}$ à $C_{15}$, le laurate d'hexyle, l'adipate de diisopropyle, l'isononanoate d'isononyle, le palmitate de 2-éthyl-hexyle, l'isostéarate d'isostéarate, des octanoates, décanoates ou ricinoléates d'alcools ou de polyalcools comme le dioctanoate de propylène glycol ; les esters hydroxylés comme le lactate d'isostéaryle, le malate de di-isostéaryle ; et les esters du pentaérythritol ;
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;
- les acides gras supérieurs tels que l'acide oléique, l'acide linoléique, l'acide linolénique ;

et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates ;

Les huiles fluorées utilisables dans la composition de l'invention sont notamment des huiles fluorosiliconées, des polyéthers fluorés, des silicones fluorées telles que décrit dans le document EP-A-847752.

La teneur en composé non volatil, non soluble dans l'eau et liquide à température ambiante est généralement de 0,01 à 25 % en poids, en particulier de 0,1 à 22 % en poids, par rapport au poids total de la composition.

## EAU ET/OU SOLVANT HYDROSOLUBLE

Selon une première variante de l'invention, les compositions proposées sont exemptes d'eau et de solvants hydrosolubles.

Selon une seconde variante de l'invention, les compositions proposées comprennent de l'eau et/ou au moins un solvant hydrosoluble en une teneur totale inférieure ou égale à 20 % en poids par rapport au poids de la composition.

Par « solvant hydrosoluble », on désigne un composé liquide à température ambiante et miscible à l'eau (miscibilité dans l'eau supérieure à 50 % en poids à 25 °C et pression atmosphérique).

Les solvants hydrosolubles éventuellement utilisables dans les compositions selon l'invention sont généralement en outre, volatils.

Parmi les solvants hydrosolubles pouvant être utilisés dans les compositions selon l'invention, on peut citer notamment les monoalcools inférieurs ayant de 1 à 5 atomes de carbone tels que l'éthanol et l'isopropanol, les glycols ayant de 2 à 8 atomes de carbone tels que l'éthylène glycol, le propylène glycol, le 1,3-butylène glycol et le dipropylène glycol, les cétones en $C_3$ et $C_4$ et les aldéhydes en $C_2$ à $C_4$.

L'eau et/ou le(s) solvant(s) hydrosoluble(s) peuvent être introduits en tant que tels dans la formulation selon l'invention ou y être incorporés par le biais, d'un ou plusieurs ingrédients constituant ladite composition. Ainsi de l'eau peut être notamment introduite dans la composition par le biais de l'introduction de latex ou de pseudolatex, c'est-à-dire de dispersion aqueuse de particules de polymère.

La présence d'eau et/ou de solvant(s) hydrosoluble(s) dans lesdites compositions peut être avantageuse pour augmenter l'adhérence de la composition sur les cils. En effet, plus le solvant non aqueux est en quantité importante, plus l'application sur les cils est glissante en raison de la nature principalement « huileuse » de la composition. La substitution partielle du solvant non aqueux par un solvant hydrosoluble peut permettre de diminuer cet effet et d'augmenter ainsi l'adhérence sur les cils. Le maquillage obtenu peut alors être plus épais.

Dans cette variante de l'invention, la teneur en eau et/ou en solvant(s) hydrosoluble(s) peut notamment être supérieure ou égale à 0,5 %, en particulier varier de 1 à 18 % et plus particulièrement de 2 à 15 % en poids par rapport au poids total de la composition.

<u>**CIRE(S)**</u>

La cire considérée dans le cadre de la présente invention est d'une manière générale un composé lipophiles, solide à température ambiante (25 °C), à changement d'état solide/liquide réversible, ayant un point de fusion supérieur ou égal à 30 °C pouvant aller jusqu'à 200 °C et notamment jusqu'à 120 °C.

En portant la cire à l'état liquide (fusion), il est possible de la rendre miscible aux huiles et de former un mélange homogène microscopiquement, mais en ramenant la température du mélange à la température ambiante, on obtient une recristallisation de la cire dans les huiles du mélange.

En particulier, les cires convenant à l'invention peuvent présenter un point de fusion supérieur ou égal à 45 °C, et en particulier supérieur ou égal à 55°C.

Au sens de l'invention, la température de fusion correspond à la température du pic le plus endothermique observé en analyse thermique (DSC) telle que décrite dans la norme ISO 11357-3 ; 1999. Le point de fusion de la cire peut être mesuré à l'aide d'un calorimètre à balayage différentiel (DSC), par exemple le calorimètre vendu sous la dénomination « MDSC 2920 » par la société TA Instruments.

Le protocole de mesure est le suivant :

Un échantillon de 5 mg de cire disposé dans un creuset est soumis à une première montée en température allant de -20 °C à 100 °C, à la vitesse de chauffe de 10 °C/minute, puis est refroidi de 100 °C à -20 °C à une vitesse de refroidissement de 10 °C/minute et enfin soumis à une deuxième montée en température allant de -20 °C à 100 °C à une vitesse de chauffé de 5 °C/minute. Pendant la deuxième montée en température, on mesure la variation de la différence de puissance absorbée par le creuset vide et par le creuset contenant l'échantillon de cire en fonction de la température. Le point de fusion du composé est la valeur de la température correspondant au sommet du pic de la courbe représentant la variation de la différence de puissance absorbée en fonction de la température.

Les cires susceptibles d'être utilisées dans les compositions selon l'invention sont choisies parmi les cires, solides, à température ambiante d'origine animale, végétale, minérale ou de synthèse et leurs mélanges.

Les cires pouvant être utilisées dans les compositions selon l'invention présentent généralement une dureté allant de 0,01 MPa à 15 MPa, notamment supérieure à 0,05 MPa et en particulier supérieure à 0,1 MPa .

La dureté est déterminée par la mesure de la force en compression mesurée à 20 °C à l'aide du texturomètre vendu sous la dénomination « TA-TX2i® » par la société RHEO, équipé d'un mobile en inox en forme de cylindre d'un diamètre de 2 mm en mesurant l'évolution de la force (force de compression ou force d'étirement) (F) en fonction du temps, pendant l'opération suivante :

Le mobile est déplacé à la vitesse de 0,1 mm/s puis pénètre dans la cire jusqu'à une profondeur de pénétration de 0,3 mm. Lorsque le mobile a pénétré dans la cire à la profondeur de 0,3 mm, le mobile est maintenu fixe pendant 1 seconde (correspondant au temps de relaxation) puis est retiré à la vitesse de 0,1 mm/s. Pendant le temps de relaxation, la force (force de compression) décroît fortement jusqu'à devenir nulle puis lors du retrait du mobile, la force (force d'étirement) devient négative pour ensuite croître à nouveau vers la valeur 0. La dureté correspond à la force de compression maximale mesurée entre la surface du mobile et la cire au moment de leur mise en contact. La valeur de cette force est exprimée en MPa.

Pour effectuer la mesure de la dureté, la cire est fondue à une température égale au point de fusion de la cire + 20 °C. La cire fondue est coulée dans un récipient de 30 mm de diamètre et de 20 mm de profondeur. La cire est recristallisée à température ambiante (25 °C) pendant 24 heures, puis la cire est conservée pendant au moins 1 heure à 20 °C avant d'effectuer la mesure de dureté.

A titre illustratif des cires convenant à l'invention, on peut notamment citer les cires hydrocarbonées comme la cire d'abeilles, la cire de lanoline, les cires d'insectes de Chine, la cire de sumac, les paraffines, certaines cires de polyéthylène et les copolymères cireux ainsi que leurs esters.

On peut aussi citer des cires obtenues par hydrogénation catalytique d'huiles animales ou végétales ayant des chaînes grasses, linéaires ou ramifiées, en $C_8$-$C_{32}$. Parmi celles-ci, on peut notamment citer l'huile de jojoba isomérisée telle que l'huile de jojoba partiellement hydrogénée isomérisée trans fabriquée ou commercialisée par la société DESERT WHALE sous la référence commerciale « Iso-Jojoba-50® », l'huile de tournesol hydrogénée, l'huile de ricin hydrogénée, l'huile de coprah hydrogénée, l'huile de lanoline hydrogénée, et le tétrastéarate de di-(triméthylol-1,1,1 propane) vendu sous la dénomination de « Hest 2T-4S® » par la société HETERENE.

On peut encore citer les cires de silicone, les cires fluorées.

On peut également utiliser les cires obtenues par hydrogénation d'huile de ricin estérifiée avec l'alcool cétylique vendues sous les dénominations de « Phytowax ricin 16L64® » et « 22L73® » par la société SOPHIM. De telles cires sont décrites dans la demande FR-A- 2792190.

Selon un mode de réalisation particulier, les compositions selon l'invention peuvent comprendre au moins une cire dite cire collante c'est-à-dire possédant un collant supérieur ou égal à 0,7 N.s et une dureté inférieure ou égale à 3,5 MPa.

L'utilisation d'une cire collante peut notamment permettre l'obtention d'une composition cosmétique qui s'applique facilement sur les fibres kératiniques, ayant une bonne accroche sur les fibres kératiniques et qui conduit à la formation d'un maquillage lisse, homogène et épaississant.

La cire collante utilisée peut posséder notamment un collant allant de 0,7 N.s à 30 N.s, en particulier supérieur ou égal à 1 N.s, notamment allant de 1 N.s à 20 N.s, en particulier supérieur ou égal à 2 N.s, notamment allant de 2 N.s à 10 N.s. et en particulier allant de 2 N.s à 5 N.s.

Le collant de la cire est déterminé par la mesure de l'évolution de la force (force de compression ou force d'étirement) en fonction du temps, à 20 °C à l'aide du texturomètre vendu sous la dénomination « TA-TX2i® » par la société RHEO, équipé d'un mobile en polymère acrylique en forme de cône formant un angle de 45°.

Le protocole de mesure est le suivant :

La cire est fondue à une température égale au point de fusion de la cire + 10 °C. La cire fondue est coulée dans un récipient de 25 mm de diamètre et de 20 mm de profondeur. La cire est recristallisée à température ambiante (25 °C) pendant 24 heures de telle sorte que la surface de la cire soit plane et lisse, puis la cire est conservée pendant au moins 1 heure à 20 °C avant d'effectuer la mesure du collant.

Le mobile du texturomètre est déplacé à la vitesse de 0,5 mm/s, puis pénètre dans la cire jusqu'à une profondeur de pénétration de 2 mm. Lorsque le mobile a pénétré dans la cire à la profondeur de 2 mm, le mobile est maintenu fixe pendant 1 seconde (correspondant au temps de relaxation) puis est retiré à la vitesse de 0,5 mm/s.

Pendant le temps de relaxation, la force (force de compression) décroît fortement jusqu'à devenir nulle puis, lors du retrait du mobile, la force (force d'étirement) devient négative pour ensuite croître à nouveau vers la valeur 0. Le collant correspond à l'intégrale de la courbe de la force en fonction du temps pour la partie de la courbe correspondant aux valeurs négatives de la force (force d'étirement). La valeur du collant est exprimée en N.s.

La cire collante pouvant être utilisée a généralement une dureté inférieure ou égale à 3,5 MPa, en particulier allant de 0,01 MPa à 3,5 MPa, notamment allant de 0,05 MPa à 3 MPa, voire encore allant de 0,1 MPa à 2,5 MPa.

La dureté est mesurée selon le protocole décrit précédemment.

Comme cire collante, on peut utiliser un (hydroxystéaryloxy)stéarate d'alkyle en $C_{20}$-$C_{40}$ (le groupe alkyle comprenant de 20 à 40 atomes de carbone), seul ou en mélange, en particulier un 12-(12'-hydroxystéaryloxy)stéarate d'alkyle en $C_{20}$-$C_{40}$, de formule (I) :

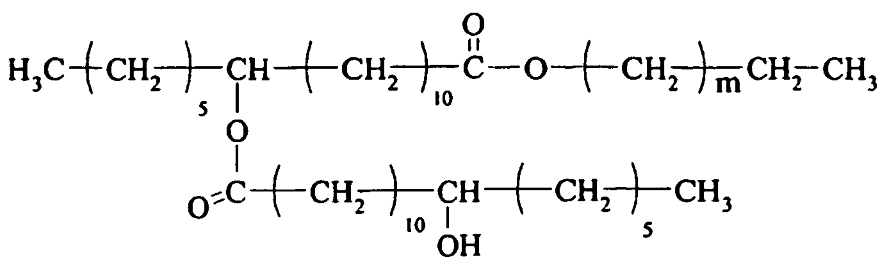

(I)

dans laquelle m est un entier allant de 18 à 38, ou un mélange de composés de formule (I).

Une telle cire est notamment vendue sous les dénominations « Kester Wax K 82 P® » et « Kester Wax K 80 P® » par la société KOSTER KEUNEN.

Les cires citées ci-dessus présentent généralement un point de fusion commençante inférieur à 45 °C.

Selon un mode de réalisation particulier des compositions selon l'invention, celles-ci peuvent comprendre au moins une cire à point de fusion commençante élevé, c'est-à-dire généralement supérieur ou égal à 45 °C, en particulier supérieur ou égal à 50 °C, voire très élevé c'est-à-dire généralement supérieur ou égal à 55 °C et en particulier supérieur ou égal à 60 °C. Le point de fusion commençante de la cire peut être mesuré à l'aide d'un calorimètre à balayage différentiel (D.S.C.), par exemple le calorimètre vendu sous la dénomination « MDSC2920® » par la société TA Instruments.

Le protocole de mesure est le même que celui décrit pour la mesure du point de fusion.

Le point de fusion commençante, désigné ci-après par l'abréviation « $pf_{com}$. » du composé correspond à la température mesurée lorsque 5 % de l'enthalpie de fusion est consommée.

Comme cires à point de fusion commençante élevé mais inférieur à 50 °C, on peut citer notamment la cire de montan ($pf_{com.}$ = 47,9 °C), l'ozokérite ($Pf_{com}$. = 46,3 °C) et la cire obtenue par hydrogénation catalytique d'huile d'olive estérifiée avec l'alcool stéarylique vendue sous la dénomination de « Phytowax Olive 18L57® » par la société SOPHIM ($pf_{com}$. = 47,4 °C).

Comme cires à point de fusion supérieur ou égal à 50 °C et inférieur à 55 °C, on peut citer notamment la cire de son de riz ($pf_{com.}$ = 51,4 °C), la cire de Candellila ($pf_{com.}$ = 50 °C) et la cire d'Ouricurry ($pf_{com.}$ = 51,4 °C).

Comme cires à point de fusion commençante très élevé pouvant être utilisées dans les compositions selon l'invention, on peut citer notamment la cire de Carnauba ($pf_{com.}$ = 63,5 °C), les cires obtenues par la synthèse de Fisher Tropsch ($pf_{com.}$ = 60,5 °C), certaines cires de polyéthylène telles que notamment celles commercialisées sous la dénomination « Performalene 655® » par la société NEW PHASE TECHNOLOGIES ou de « Polyethylene AC 540® » par la société HONEYWELL, de « Polywax 2000 T-6® » par la société PETROLITE ($pf_{com.}$ = 125 °C), ou de « PED 191® » et « Epolene N-14® » par la société EASTMAN KODAK (respectivement $pf_{com.}$ = 120 °C et 106 °C) et certaines cires microcristallines telles que celles commercialisées sous les dénominations de « Tisco Wax 88® » par la société TISCO ou de « Microwax HW® » par la société PARAMELT.

Comme cires à point de fusion commençante très élevé, on peut également citer des cires obtenues par hydrogénation catalytique d'huiles animales ou végétales ayant des chaînes grasses, linéaires ou ramifiées, en $C_8$-$C_{32}$ telles que l'huile de jojoba hydrogénée ($pf_{com.}$ = 63,2 °C) et le tétrabéhénate de di-(triméthylol-1,1,1 propane) vendu sous la dénomination de « Hest 2T-4B® » par la société HETERENE ($pf_{com.}$ = 61,8 °C).

La teneur en cire ayant un point de fusion commençante élevé, voire très élevé, dans les compositions selon l'invention peut être particulièrement importante et être notamment supérieure ou égale à 20 % en poids.

Dans la présente invention, on utilise des cires fournies sous forme de petites particules ayant une dimension de l'ordre de 0,5 à 30 micromètres, en particulier de 1 à 20 micromètres, et plus particulièrement de 5 à 10 micromètres, désignées par la suite par l'expression « micro cires ». A des fins de distinction, les cires mises en oeuvre selon l'invention sous forme de fragments de dimension plus élevée sont par la suite désignées par l'expression « cires de type traditionnel ».

Comme micro cires pouvant être utilisées dans les compositions selon l'invention, on peut citer notamment les micro cires de carnauba telles que celle commercialisée sous la dénomination de « MicroCare 350® » par la société MICRO POWDERS, les micro cires de cire synthétique telles que celle commercialisée sous la dénomination de « MicroEase 114S® » par la société MICRO POWDERS, les micro cires constituées d'un mélange de cire de carnauba et de cire de polyéthylène telles que celles commercialisées sous les dénominations de « Micro Care 300® » et « 310® » par la société MICRO POWDERS, les micro cires constituées d'un mélange de cire de carnauba et de cire synthétique telles que celle commercialisée sous la dénomination « Micro Care 32® » par la société MICRO POWDERS, les micro cires de polyéthylène telles que celles commercialisées sous les dénominations de « Micropoly 200® », « 220® », « 220L® » et « 250S® » par la société MICRO POWDERS et les micro cires de polytétrafluoroéthylène telles que celles commercialisées sous les dénominations de « Microslip 519® » et « 519 L® » par la société MICRO POWDERS.

Parmi les micro cires citées précédemment, certaines telles que par exemple la micro cire de carnauba, la micro cire de cire synthétique « MicroEase 114S® » ou la micro cire constituée d'un mélange de cire de carnauba et de cire synthétique « MicroCare 325® »présentent un point de fusion commençante supérieur ou égal à 45 °C.

Dans la composition selon l'invention, on peut bien entendu utiliser un mélange de cires et notamment utiliser une ou plusieurs cires de type traditionnel telles que notamment une cire collante et/ou une cire à point de fusion commençante supérieur ou égal à 45 °C, et une ou plusieurs cires dites micro cires.

La composition selon l'invention contient une teneur totale en cires supérieure à 25 % en poids par rapport au poids total de la composition.

En particulier, la teneur totale en cire peut aller de 25 à 70 % en poids, notamment de 25 à 65 % et en particulier de 25 à 60%, voire de 25 à 55% en poids par rapport au poids total de la composition.

La cire ou le mélange de cires est présent, dans les compositions selon l'invention, sous la forme d'une dispersion de particules dans le milieu solvant non aqueux.

Les particules de cire peuvent présenter des formes variées. Elles peuvent , notamment être sensiblement

sphériques.

Une observation au microscope d'un échantillon de la composition, à température ambiante, montre une bonne dispersion des particules de cires dans ledit milieu, avec peu ou pas d'agrégat de ces particules, voire une répartition des particules sensiblement identique dans toutes les directions.

## Polymère(s) soluble(s) dans le milieu solvant non aqueux et avant au moins une partie cristallisable

La composition selon l'invention comprend au moins un polymère soluble dans le milieu solvant non aqueux et ayant au moins une partie cristallisable.

Par « polymère soluble dans ledit solvant milieu non aqueux », on entend un polymère qui, lorsqu'il est introduit seul en une quantité en matière sèche au moins supérieure à 0,01% en poids et pour une quantité correspondante à celle envisagée pour la composition finale désirée, est soluble dans ledit milieu solvant non aqueux à température ambiante, généralement de l'ordre de 25 °C, et sous pression atmosphérique (750 mm de Hg, soit $10^5$ Pa).

Au sens de la présente invention, on désigne sous le terme de « polymère », un composé possédant au moins deux motifs de répétition, notamment au moins trois motifs de répétition, en particulier au moins dix motifs de répétition, voire au moins quinze motifs de répétition. Le polymère conforme à l'invention est généralement composé d'au moins deux motifs répétitifs de nature différente (copolymère). Les polymères utilisés dans l'invention sont généralement d'origine synthétique et sont caractérisés par des masses molaires allant de 200 à 1000 000 g/mol, en particulier de 500 à 500 000 g/mol et plus particulièrement de 1000 à 300 000 g/mol.

Plus précisément, les polymères utilisés dans la présente invention sont des copolymères solubilisés et non cristallisés dans le milieu à température ambiante et comportent obligatoirement au moins une partie cristallisable désignée A et au moins une partie non cristallisable dite amorphe, désignée B.

De part cette structure spécifique, ils possèdent avantageusement à la fois une affinité pour les cires grâce à la partie A et une affinité pour le solvant grâce à la partie B et participent donc à ce titre efficacement à la dispersion des cires dans le milieu solvant non aqueux.

La partie cristallisable des polymères utilisés dans la présente invention représente au moins 5%, en particulier au moins 10% et au plus 50%, et plus particulièrement de 30 à 50% en poids du poids total de chaque polymère.

La partie cristallisable A d'un copolymère selon l'invention peut être figurée par une chaîne pendante liée au squelette dudit polymère et/ou une séquence intégrée directement dans ce squelette et/ou au moins une chaîne terminale. Ces copolymères peuvent être de toute structure chimique : copolymères statistiques, séquences, greffés et/ou dendrimères.

De la même façon, la partie amorphe d'un copolymère selon l'invention peut être figurée par une chaîne pendante liée au squelette dudit copolymère et/ou une séquence intégrée directement dans ce squelette et/ou au moins une chaîne terminale.

On entend désigner au sens de l'invention par le terme ou l'expression :

- « partie cristallisable A », un enchaînement d'au moins 5 motifs de répétition et tel que si l'on prenait l'homopolymère correspondant à ce motif de répétition, il serait caractérisé par un taux de cristallinité supérieur à 30%,
- « partie amorphe B », un enchaînement d'au moins 5 motifs de répétition et tel que si l'on prenait l'homopolymère correspondant à ce motif de répétition, il serait caractérisé par un taux de cristallinité inférieur à 5%, voire nul,
- « séquence intégrée au squelette », un groupement d'atomes constitué par la répétition d'une unité monomère, faisant partie de la chaîne principale du polymère,
- « chaîne pendante ou groupement latéral », un groupement d'atomes figurant une ramification au niveau du squelette du polymère, et
- « chaîne terminale » un groupement d'atome situé à l'une au moins des extrémités du squelette.

### a) Les copolymères statistiques

Les copolymères statistiques sont de préférence des polymères à chaînes pendantes cristallisables qui comportent des motifs résultant de la polymérisation d'au moins deux monomères dont l'un au moins possède une chaîne latérale hydrophobe cristallisable appelée X qui peut être représenté par la formule II :

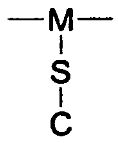 (II)

avec M représentant un atome du squelette polymérique, S représentant un espaceur, C représentant un groupe cristallisable.

Les chaînes « -S-C » cristallisables peuvent être aliphatiques ou aromatiques, linéaires, ramifiées ou cycliques, éventuellement fluorées ou perfluorées. « S » représente notamment un groupe $(CH_2)_n$ ou $(CH_2CH_2O)_n$ ou $(CH_2O)$, linéaire ramifié ou cyclique, avec n étant un nombre entier allant de 0 à 22. En particulier, « S » est un groupe linéaire. En particulier, « S » et « C » sont différents.

Lorsque les chaînes « -S-C » cristallisables sont des chaînes aliphatiques hydrocarbonées, elles comportent des chaînes alkyles hydrocarbonées ayant au moins 11 atomes de carbone et au plus 40 atomes de carbone et en particulier au plus 24 atomes de carbone. Il s'agit notamment de chaînes aliphatiques ou chaînes alkyles possédant au moins 12 atomes de carbone et en particulier, il s'agit de chaînes alkyles en $C_{12}$-$C_{24}$-Lorsqu'il s'agit de chaînes alkyles fluorées ou perfluorées, elles comportent au moins 6 atomes de carbone fluorés et notamment au moins 11 atomes de carbone dont au moins 6 atomes de carbone sont fluorés.

Comme exemple de polymères à chaîne(s) pendante(s) cristallisable(s), on peut citer ceux comportant des motifs résultant de la polymérisation d'un ou plusieurs monomères suivants : les (méth)acrylates d'alkyle saturé avec le groupe alkyle en $C_{12}$-$C_{24}$, les (méth)acrylates de perfluoroalkyle avec un groupe alkyle perfluoro en $C_{12}$-$C_{15}$, les N-alkyl (méth)acrylamides avec le groupe alkyle en $C_{12}$ à $C_{24}$ avec ou sans atome de fluor, les esters vinyliques ou allyliques à chaînes alkyle ou perfluoro (alkyle) avec le groupe alkyle en $C_{12}$ à $C_{24}$ (avec au moins 6 atomes de fluor pour une chaîne perfluoro alkyle), les éthers vinyliques à chaînes alkyle ou perfluoro (alkyle) avec le groupe alkyle en $C_{12}$ à $C_{24}$ et au moins 6 atomes de fluor pour une chaîne perfluoro alkyle, les alpha-oléfines en $C_{12}$ à $C_{24}$ comme par exemple l'octadécène, les para-alkyl styrènes avec un groupe alkyle comportant de 12 à 24 atomes de carbone, leurs mélanges.

A titre illustratif de ces polymères utilisables dans la présente invention, on citera les copolymères (méth) acrylates d'alkyles linéaires et saturés en $C_{12}$ à $C_{30}$, constituant la partie cristallisable A et (méth)acrylates d'alkyles en $C_4$ à $C_{10}$ linéaires, ou en $C_4$ à $C_{30}$ ramifiés ou cycliques et/ou insaturés, constituant la partie amorphe B.

Parmi les copolymères d'esters vinyliques à groupes alkyles linéaires et saturés en $C_{12}$ à $C_{30}$ constituant la partie cristallisable A et d'esters vinyliques à groupes alkyles en $C_4$ à $C_{10}$ linéaires ou en $C_4$ à $C_{30}$ ramifiés ou cycliques et/ou insaturés constituant la partie amorphe B, on peut citer en particulier les copolymères d'acétate de vinyle et de stéarate de vinyle ou de stéarate d'allyle tel que le copolymère de stéarate d'allyle et d'acétate de vinyle vendu sous la dénomination de « Mexomère PQ® » par la société CHIMEX.

Lorsque les polymères résultent d'une polycondensation, les chaînes cristallisables hydrocarbonées et/ou fluorées telles que définies ci-dessus, sont portées par un monomère qui peut être un diacide, un diol, une diamine ou un di-isocyanate.

**b) Les copolymères séquencés**

Ces copolymères sont constitués d'au moins deux types de séquences de nature chimique différente dont l'une est cristallisable et constitue la partie A. Dans le cas des copolymères séquencés, l'une au moins des séquences amorphes B doit être soluble dans le milieu.

On peut citer par exemple :

- des copolymères séquencés d'oléfine ou de cyclooléfine à chaîne cristallisable comme ceux issus de la polymérisation séquencée :

  - de cyclobutène, cyclohexène, cyclooctène, norbornène (c'est-à-dire bicyclo(2,2,1 1)heptène-2), 5-méthylnorbomène, 5-éthylnorbomène, 5,6-diméthylnorbomène, 5,5,6-triméthyl norbomène, 5-éthylidène-norbornène, 5-phényl-norbonène, 5-benzylnorbornène, 5-vinyl norbornène, 1,4,5,8-diméthano-1,2,3,4,4a,5,8a-octahydronaphtalène, dicyclopentadiène ou de leurs mélanges,
  - avec l'éthylène, propylène, 1-butène, 3-méthyl-1-butène, 1-hexène, 4-méthyl-1-pentène, 1-octène, 1-décène, 1-éicosène ou leurs mélanges,

- les copolymères séquencés poly(butylènetéréphtalate)-b-poly(isoprène) hydrogénés séquencés ou multiséquencés, cités dans l'article "Study of morphological and mechanical properties of PP/PBT" de B. Boutevin et al., Polymer Bulletin, 34,117-123 (1995),
- les copolymères séquencés poly(éthylène)-b-copoly(éthylène/propylène) cités dans les articles "Morphology of semi-crystalline block copolymers of ethylene-(ethylene-alt-propylene)" de P. Rangarajan et al., Macromolecules, 26, 4640-4645 (1993) et " Polymer agregates with crystalline cores : the system poly(ethylene)-poly(ethylene-propylene)" de P. Richter et al., Macromolécules, 30, 053-1068 (1997), et
- les copolymères séquencés poly(éthylène)-b-poly(éthyléthylène) cités dans l'article général "Cristallization in block copolymers" de I.W. Hamley, Advances in Polymer Science, vol 148, 113-137 (1999).

Ces polymères peuvent présenter une unique séquence cristallisable ou une répétition de séquence cristallisables. Dans ce dernier cas, ces séquences cristallisables peuvent être de nature chimique identique ou différente.

**c) Les copolymères à séquences cristallisables terminales**

On peut citer par exemple dans cette catégorie :

- les polycondensats de type polyamide résultant de la condensation entre (α) au moins un acide choisi parmi les acides dicarboxyliques comprenant au moins 32 atomes de carbone tels que les acides gras dimères et (β) un alkylène diamine et en particulier l'éthylène diamine, dans lequel le polymère polyamide comprend au moins un groupe acide carboxylique terminal estérifié ou amidifié avec au moins un mono alcool ou une mono amine comprenant de 12 à 30 atomes de carbone linéaires et saturés, et en particulier, les copolymères d'éthylène diamine/dilinoléate de stéaryle tel que celui commercialisé sous la dénomination « Uniclear 100 VG® » par la Société ARIZONA CHEMICAL ; et
- les polycondensats polyesters lipophiles dont les extrémités sont estérifiées par un acide ou un alcool cristallisable constitué d'une chaîne carbonée linéaire saturée en $C_{12}$ à $C_{30}$, et en particulier, l'acide 12-polyhydroxystéarique dont au moins une des extrémités est estérifiée par l'acide stéarique, tel que le « Solsperse 21000® » commercialisé par la société AVECIA.

A titre complémentaire et illustratif des copolymères selon l'invention, on peut citer en particulier les copolymères éthylène/acétate de vinyle, les copolymères éthylène/anhydride maléique, les copolymères séquences butadiène hydrogéné/isoprène et les terpolymères éthylènelanhydride maléique/acétate de vinyle.
Le polymère soluble dans le milieu solvant non aqueux et ayant au moins une partie cristallisable ou un mélange de tels polymères peut être présent dans la composition selon l'invention en une proportion allant de 0,01 % à 30 %, notamment de 0,1 à 20 % et en particulier de 1 à 10 % en poids par rapport au poids total de la composition.

**Polymère filmogène**

La composition selon l'invention peut comprendre selon un mode de réalisation particulier au moins un polymère filmogène.
Dans la présente invention, on entend par « polymère filmogène », un polymère apte à former à lui seul ou en présence d'un agent auxiliaire de filmification, un film continu et adhérent sur un support, notamment sur les matières kératiniques.
Dans la présente invention, sont classés dans cette catégorie, des polymères généralement liposolubles comportant moins de 30% en poids de partie cristallisable dans les conditions de l'invention et en particulier n'en contenant pas.
Parmi les polymères filmogènes utilisables dans la composition de la présente invention, on peut citer les polymères synthétiques, de type radicalaire ou de type polycondensat, les polymères d'origine naturelle, et leurs mélanges.
A titre d'exemple de polymère liposoluble, on peut citer les copolymères d'ester vinylique (le groupe vinylique étant directement relié à l'atome d'oxygène du groupe ester et l'ester vinylique ayant un radical hydrocarboné saturé, linéaire ou ramifié, de 1 à 24 atomes de carbone, lié au carbonyle du groupe ester) et d'au moins un autre monomère qui peut être un ester vinylique (différent de l'ester vinylique déjà présent), un alkylvinyléther (dont le groupe alkyle comporte de 2 à 24 atomes de carbone), ou un ester allylique ou méthallylique (ayant un radical hydrocarboné saturé, linéaire ou ramifié, de 1 à 24 atomes de carbone, lié au carbonyle du groupe ester). Ces copolymères peuvent être réticulés à l'aide de réticulants qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.

Comme exemples de ces copolymères, on peut citer les copolymères : acétate de vinyle/laurate de vinyle, propionate de vinyle/laurate d'allyle, propionate de vinyle/laurate de vinyle, et diméthyl-2, 2 pentanoate d'allyle/laurate de vinyle.

Comme polymères filmogènes liposolubles, on peut également citer les homopolymères liposolubles, et en particulier ceux résultant de l'homopolymérisation d'esters vinyliques ayant de 9 à 22 atomes de carbone ou d'acrylates ou de méthacrylates d'alkyle, les radicaux alkyles ayant de 2 à 24 atomes de carbone.

Comme exemples d'homopolymères liposolubles, on peut citer notamment: les polylaurate de vinyle et le poly (méth)acrylates de lauryle, ces poly(méth)acrylates pouvant être réticulés à l'aide de diméthacrylate de l'éthylène glycol ou de tétraéthylène glycol.

Les copolymères et homopolymères liposolubles définis précédemment sont connus et notamment décrits dans la demande FR-A-2232303 ; ils peuvent avoir un poids moléculaire moyen en poids allant de 2.000 à 500.000 et en particulier de 4.000 à 200.000.

Comme polymères filmogènes liposolubles utilisables dans l'invention, on peut également citer les polyalkylènes et notamment les copolymères d'alcènes en $C_2$ à $C_{20}$, comme le polybutène, les alkylcelluloses avec un radical alkyle linéaire ou ramifié, saturé ou non en $C_1$ à $C_8$ comme l'éthylcellulose et la propylcellulose, les copolymères de la vinylpyrolidone (VP) et notamment les copolymères de la vinylpyrrolidone et d'alcène en $C_2$ à $C_{40}$ et en particulier en $C_3$ à $C_{20}$. A titre d'exemple de copolymères de VP utilisables dans l'invention, on peut citer les copolymères de VP/acétate vinyle, VP/méthacrylate d'éthyle, la polyvinylpyrolidone (PVP) butylée, VP/méthacrylate d'éthyle/acide méthacrylique, VP/eicosène, VP/hexadécène, VP/triacontène, VP/styrène et VP/acide acrylique/méthacrylate de lauryle.

Le polymère filmogène peut être également présent dans la composition sous la forme de particules en dispersion dans une phase aqueuse ou dans une phase solvant non aqueuse, connue généralement sous le nom de latex ou pseudolatex. Les techniques de préparation de ces dispersions sont bien connues de l'homme du métier.

Comme dispersion aqueuse de polymère filmogène, on peut utiliser les dispersions acryliques vendues sous les dénominations « Neocryl XK-90® », « Neocryl A-1070® », « Neocryl A-1090® », « Neocryl BT-62® », « Neocryl A-1079® » et « Neocryl A-523® » par la société AVECIA-NEORESINS, « Dow Latex 432® » par la société DOW CHEMICAL, « Daitosol 5000 AD® » par la société DAITO KASEY KOGYO; ou encore les dispersions aqueuses de polyuréthane vendues sous les dénominations « Neorez R-981® » et « Neorez R-974® » par la société AVECIA-NEORESINS, les « Avalure UR-405® », « Avalure UP-410® », « Avalure UR-425® », « Avalure UR-450® », « Sancure 875® », « Sancure 861®», « Sancure 878® » et « Sancure 2060® » par la société GOODRICH, « Impranil 85® » par la société BAYER, « Aquamere H-1511® » par la société HYDROMER ; les suifopolyesters vendus sous le nom de marque « Eastman AQ® » par la société EASTMAN CHEMICAL PRODUCTS, les dispersions vinyliques comme le « Mexomère PAM » et aussi les dispersions acryliques dans l'isododécane comme le « Mexomère PAP » par la société CHIMEX.

Le polymère filmogène peut être présent dans la composition selon l'invention en une teneur en matières sèches allant de 0,1 % à 30 % en poids par rapport au poids total de la composition, en particulier de 0,5 % à 25 % en poids, et plus particulièrement de 1 % à 20 % en poids.

La composition selon l'invention peut comprendre un agent plastifiant favorisant la formation d'un film avec le polymère filmogène. Un tel agent plastifiant peut être choisi parmi tous les composés connus de l'homme du métier comme étant susceptibles de remplir la fonction recherchée.

## Matière colorante

La composition selon l'invention peut également comprendre au moins une matière colorante comme les matières pulvérulentes, les colorants liposolubles, les colorants hydrosolubles.

Les matières colorantes pulvérulentes peuvent être choisies parmi les pigments et les nacres.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les colorants liposolubles sont par exemple le rouge Soudan, le D&C Red 17, le D&C Green 6, le β-caroténe, l'huile de soja, le brun Soudan, le D&C Yellow 11, le D&C Violet 2, le D&C Orange 5, le jaune quinoléine, le rocou.

Ses matières colorantes peuvent être présentes en une teneur allant de 0,01 à 30 % en poids par rapport au poids total de la composition.

**Charges**

La composition selon l'invention peut en outre comprendre au moins une charge.

Les charges peuvent être choisies parmi celles bien connues de l'homme du métier et couramment utilisées dans les compositions cosmétiques. Les charges peuvent être minérales ou organiques, lamellaires ou sphériques. On peut citer le talc, le mica, la silice, le kaolin, les poudres de polyamide comme le Nylon® commercialisé sous la dénomination « d'Orgasol® » par la société ATOCHEM, de poly-β-alanine et de polyéthylène, les poudres de polymères de tétrafluoroéthylène comme le Téflon®, la lauroyl-lysine, l'amidon, le nitrure de bore, les micro sphères creuses polymériques expansées telles que celles de chlorure de polyvinylidène/acrylonitrile comme celles commercialisées sous la dénomination « d'Expancel® » par la société NOBEL INDUSTRIE, les poudres acryliques telles que celles commercialisées sous la dénomination « Polytrap® » par la société DOW CORNING, les particules de polyméthacrylate de méthyle et les microbilles de résine de silicone (« Tospearls® » de TOSHIBA, par exemple), le carbonate de calcium précipité, le carbonate et l'hydrocarbonate de magnésium, l'hydroxyapatite, les microsphères de silice creuses (« Silica Beads® » de MAPRECOS), les microcapsules de verre ou de céramique, les savons métalliques dérivés d'acides organiques carboxyliques ayant de 8 à 22 atomes de carbone, et en particulier de 12 à 18 atomes de carbone, par exemple le stéarate de zinc, de magnésium ou de lithium, le laurate de zinc, le myristate de magnésium.

Les charges peuvent représenter de 0,1 à 25 %, en particulier de 1 à 20 % en poids par rapport au poids total de la composition.

La composition de l'invention peut comprendre, en outre, tout additif cosmétiquement acceptable en particulier choisi parmi ceux usuellement utilisés en cosmétique tels que les antioxydants, les conservateurs, les parfums, les neutralisants, les plastifiants, les épaississants ou gélifiants, les fibres, les actifs cosmétiques et leurs mélanges.

Les gélifiants utilisables dans les compositions selon l'invention sont généralement lipophiles et peuvent être organiques ou minéraux, polymériques ou moléculaires.

Comme gélifiant lipophile minéral, on peut citer les argiles éventuellement modifiées comme les hectorites modifiées par un chlorure d'ammonium d'acide gras en $C_{10}$ à $C_{22}$, comme l'hectorite modifiée par du chlorure de distéaryl di-méthyl ammonium telle que, par exemple, celle commercialisée sous la dénomination de « Bentone 38V® **»** par la société ELEMENTIS.

On peut également citer la silice pyrogénée éventuellement traitée hydrophobe en surface dont la taille des particules est inférieure à 1 μm. Il est en effet possible de modifier chimiquement la surface de la silice, par réaction chimique générant une diminution du nombre de groupes silanol présents à la surface de la silice. On peut notamment substituer des groupes silanol par des groupements hydrophobes : on obtient alors une silice hydrophobe. Les groupements hydrophobes peuvent être :

- des groupements triméthylsiloxyle, qui sont notamment obtenus par traitement de silice pyrogénée en présence de l'hexaméthyldisilazane. Des silices ainsi traitées sont dénommées « Silica silylate » selon le CTFA (6ème édition, 1995). Elles sont par exemple commercialisées sous les références « Aerosil R812® » par la société DEGUSSA, « CAB-O-SIL TS-530® » par la société CABOT,
- des groupements diméthylsilyloxyle ou polydiméthylsiloxane, qui sont notamment obtenus par traitement de silice pyrogénée en présence de polydiméthylsiloxane ou du diméthyldichlorosilane. Des silices ainsi traitées sont dénommées « Silica diméthyl silylate » selon le CTFA (6ème édition, 1995). Elles sont par exemple commercialisées sous les références « Aerosil R972® », et « Aerosil R974® » par la société DEGUSSA, « CAB-O-SIL TS-610® » et « CAB-O-SIL TS-720® » par la société CABOT.

La silice pyrogénée hydrophobe présente en particulier une taille de particules pouvant être nanométrique à micrométrique, par exemple allant d'environ de 5 à 200 nm.

Les gélifiants lipophiles organiques polymériques sont par exemple les organopolysiloxanes élastomériques partiellement ou totalement réticulés, de structure tridimensionnelle, comme ceux commercialisés sous les dénominations de « KSG6® », « KSG16® » et de « KSG 18® » par la société SHIN-ETSU, de « Trefil E-505C® » et «Trefil E-506C® » par la société DOW-CORNING, de « Gransil SR-CYC® », « SR DMF10® », «SR-DC556®», «SR 5CYC gel® », «SR DMF 10 gel® » et de « SR DC 556 gel®» par la société GRANT INDUSTRIES, de « SF 1204®» et de « JK 113® » par la société GENERAL ELECTRIC ; l'éthylcellulose comme celle vendue sous la dénomination d'« Ethocel® » par la société DOW CHEMICAL ; les galactommananes comportant de un à six, et en particulier de deux à quatre, groupes hydroxyle par ose, substitués par une chaîne alkyle saturée ou non, comme la gomme de guar alkylée par des chaînes alkyle en $C_1$ à $C_6$, et en particulier en $C_1$ à $C_3$ et leurs mélanges. Les copolymères séquencés de type « dibloc » ou « tribloc » du type polystyrène/polyisoprène, polystyrène/polybutadiène tels que ceux commercialisés sous la dénomination de « Luvitol HSB® » par la société BASF, du type polystyrène/copoly(éthylène-propylène) tels que ceux commercialisés sous la dénomination de « Kraton® » par la société SHELL CHEMICAL CO ou encore du type polystyrène/copoly(éthylène-butylène).

Parmi les gélifiants pouvant être utilisés dans les compositions selon l'invention, on peut encore citer les esters de dextrine et d'acide gras, tels que les palmitates de dextrine, notamment tels que ceux commercialisés sous les dénominations de « Rheopearl TL® » ou « Rheopearl KL® » par la société CHIBA FLOUR.

La composition selon l'invention peut en outre comprendre des fibres qui permettent une amélioration de l'effet allongeant.

Par « fibre », il faut comprendre un objet de longueur L et de diamètre D tel que L soit très supérieur à D, D étant le diamètre du cercle dans lequel s'inscrit la section de la fibre. En particulier, le rapport L/D (ou facteur de forme) est choisi dans la gamme allant de 3,5 à 2500, en particulier de 5 à 500, et plus particulièrement de 5 à 150.

Les fibres utilisables dans la composition de l'invention peuvent être des fibres d'origine synthétique ou naturelle, minérale ou organique. Elles peuvent être courtes ou longues, unitaires ou organisées par exemple tressées, creuses ou pleines. Leur forme peut être quelconque et notamment de section circulaire ou polygonale (carrée, hexagonale ou octogonale) selon l'application spécifique envisagée. En particulier, leurs extrémités sont épointées et/ou polies pour éviter de se blesser.

En particulier, les fibres ont une longueur allant de 1 μm à 10 mm, en particulier de 0,1 mm à 5 mm et plus particulièrement de 1 mm à 3,5 mm. Leur section peut être comprise dans un cercle de diamètre allant de 2 nm à 500 μm, en particulier allant de 100 nm à 100 μm et plus particulièrement de 1 μm à 50 μm. Le poids ou titre des fibres est souvent donné en denier ou décitex et représente le poids en gramme pour 9 km de fil. Les fibres selon l'invention peuvent en particulier avoir un titre choisi dans la gamme allant de 0,15 à 30 deniers et notamment de 0,18 à 18 deniers.

Les fibres peuvent être celles utilisées dans la fabrication des textiles et notamment des fibres de soie, de coton, de laine, de lin, des fibres de cellulose (notamment extraites notamment du bois, des légumes ou des algues), de rayonne, de polyamide (« Nylon® »), de viscose, d'acétate notamment d'acétate de rayonne, de poly-(p-phénylène-téréphtalamide) (ou d'aramide) notamment de « Kevlar® », de polymère acrylique notamment de polyméthacrylate de méthyle ou de poly 2-hydroxyéthyl méthacrylate, de polyoléfine et notamment de polyéthylène ou de polypropylène, de verre, de silice, de carbone notamment sous forme graphite, de polytétrafluoroéthylène (comme le « Téflon® »), de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères tels que ceux mentionnés ci-avant, comme des fibres de polyamide/polyester.

On peut aussi utiliser les fibres utilisées en chirurgie comme les fibres synthétiques résorbables préparées à partir d'acide glycolique et caprolactone (« Monocryl® » de chez JOHNSON & JOHNSON) ; les fibres synthétiques résorbables du type copolymère d'acide lactique et d'acide glycolique (« Vicryl® » de chez JOHNSON & JOHNSON) ; les fibres de polyester téréphtalique (« Ethibond® » de chez JOHNSON & JOHNSON) et les fils d'acier inoxydable (« Acier® » de chez JOHNSON & JOHNSON).

Par ailleurs, les fibres peuvent être traitées ou non en surface, enrobées ou non. Comme fibres enrobées utilisables dans l'invention, on peut citer des fibres de polyamides enrobées de sulfure de cuivre pour un effet anti-statique (par exemple le « R-STAT® » de chez RHODIA) ou un autre polymère permettant une organisation particulière des fibres (traitement de surface spécifique) ou traitement de surface induisant des effets de couleurs/hologrammes (fibre « Lurex® » de chez SILDOREX, par exemple).

En particulier, on utilise des fibres d'origine synthétiques et en particulier des fibres organiques, comme celles utilisées en chirurgie. Avantageusement, on peut utiliser des fibres insolubles dans l'eau.

Les fibres utilisables dans la composition selon l'invention sont en particulier des fibres de polyamide, de cellulose, de poly-p-phénylène téréphtalamide ou de polyéthylène. Leur longueur (L) peut aller de 0,1 m à 5 mm, en particulier de 0,25 mm à 1,6 mm et leur diamètre moyen peut aller de 1 μm à 50 μm. En particulier, on peut utiliser les fibres de polyamide commercialisées par les Etablissements P. Bonte sous le nom de « Polyamide 0.9 Dtex 3 mm® », ayant un diamètre moyen de 6 μm, un titre d'environ 0,9 dtex et une longueur allant de 0,3 mm à 5 mm. On peut aussi utiliser les fibres de celluloses (ou de rayonne) ayant un diamètre moyen de 50 μm et une longueur allant de 0,5 mm à 6 mm comme celles vendues sous le nom de « Natural rayon flock fiber RC1BE - N003 - M04® » par la société CLAREMONT FLOCK. On peut également utiliser des fibres de polyéthylène comme celles vendues sous le nom de « Shurt Stuff 13 099 F® » par la société MINI FIBERS.

La composition selon l'invention peut également comprendre des fibres dites « rigides », par opposition aux fibres citées précédemment, qui ne sont pas des fibres rigides.

Les fibres rigides, initialement sensiblement droites, lorsqu'elles sont placées dans un milieu dispersant, ne voient pas leur forme sensiblement modifiée, ce qui se traduit par la condition angulaire définie ci-après, reflétant une forme que l'on peut qualifier de toujours, sensiblement droite, linéaire. Cette condition d'angle reflète la rigidité des fibres qui peut difficilement être exprimée par un autre paramètre pour des objets ayant une taille aussi faible que les fibres rigides.

La rigidité des fibres se traduit par la condition angulaire suivante : avantageusement, au moins 50 % en nombre, en particulier au moins 75 % en nombre, et plus particulièrement au moins 90 % en nombre des fibres sont telles que l'angle formé entre la tangente à l'axe central longitudinal de la fibre et la droite reliant ladite extrémité au

point sur l'axe central longitudinal de la fibre correspondant à la moitié de la longueur de la fibre soit inférieur à 15° et l'angle formé entre la tangente à l'axe central longitudinal de la fibre en un point situé à mi-longueur de la fibre et la droite reliant l'une des extrémités au point sur l'axe central longitudinal de la fibre correspondant à la moitié de la longueur de fibre soit inférieur ou égal à 15°, pour une même longueur de fibre allant de 0,8 mm à 5 mm, en particulier allant de 1 mm à 4 mm, plus particulièrement allant de 1 mm à 3 mm, voire de 2 mm.

Avantageusement, l'angle, mentionné ci-dessus, est mesuré aux deux extrémités de la fibre et en un point situé à mi-longueur de la fibre, en d'autres termes, on effectue dans ce cas trois mesures et la moyenne des angles mesurés est inférieure ou égale à 15°.

Notamment, la tangente, en tout point de la fibre, forme un angle inférieur à 15°.

Dans la présente demande, l'angle formé par la tangente en un point de la fibre est l'angle formé entre la tangente à l'axe central longitudinal de la fibre audit point de la fibre et la droite reliant l'extrémité de la fibre la plus proche dudit point au point sur l'axe central longitudinal de la fibre correspondant à la moitié de la longueur de la fibre.

Généralement, les fibres rigides utilisables dans la composition selon l'invention ont la même longueur de fibre ou une longueur sensiblement identique.

Plus précisément lorsque l'on observe au microscope, avec un objectif permettant un grossissement de 2,5 et avec une vision plein champ, un milieu dans lequel sont dispersées les fibres rigides à une concentration des fibres de 1 % en poids, un nombre majoritaire de fibres rigides, c'est-à-dire au moins 50 % en nombre des fibres rigides, en particulier au moins 75 % en nombre des fibres rigides, et plus particulièrement au moins 90 % en nombre des fibres rigides, doivent satisfaire à la condition angulaire définie plus haut. La mesure conduisant à la valeur de l'angle est effectuée pour une même longueur de fibres, cette longueur est comprise dans la gamme allant de 0,8 mm à 5 mm, en particulier de 1 à 4 mm, plus particulièrement de 1 à 3 mm, voire de 2 mm.

Le milieu dans lequel est réalisée l'observation est un milieu dispersant assurant une bonne dispersion des fibres rigides, par exemple de l'eau, un gel aqueux d'argile ou de polyuréthane associatif. On peut même réaliser une observation directe de la composition contenant les fibres rigides. Un échantillon de la composition ou de la dispersion préparée est placé entre lame et lamelle pour l'observation au microscope avec un objectif permettant un grossissement de 2,5 et avec une vision plein champ. La vision plein champ permet de voir les fibres dans leur intégralité.

Les fibres rigides peuvent être choisies parmi les fibres d'un polymère synthétique choisi parmi les polyesters, les polyuréthanes, les polymères acryliques, les polyoléfines, les polyamides, en particulier les polyamides non aromatiques et les polyimides-amides aromatiques.

Comme exemples de fibres rigides, on peut citer les fibres :

- de polyesters, telles que celles obtenues par découpe de fils vendus sous les dénominations «FIBRE 255-100-R11-242T TAILLE 3 MM (section octalobée)® », « FIBRE 265-34-R11-56T TAILLE 3 MM (section ronde)® », « FIBRE COOLMAX 50-34-591 TAILLE 3 MM (section tétralobée)® » par la société DUPONT DE NEMOURS ;
- de polyamide, telles que celles vendues sous les dénominations «TRILOBAL NYLON 0.120-1.8 DPF® » ; « TRILOBAL NYLON 0.120-18 DPF® » ; « NYLON 0.120-6 DPF® » par la société CELLUSUEDE PRODUCTS ; ou obtenues par découpe de fils vendus sous la dénomination « FIBRE NOMEX BRAND 430 TAILLE 3 MM® » par la société DUPONT DE NEMOURS ;
- de polyimide-amide telles que celles vendues sous les dénomination « KERMEL® », « KERMEL TECH®» par la société RHODIA ;
- de poly-(p-phénylène-téréphtalamide) (ou d'aramide) notamment vendues sous la dénomination « Kevlar® » par la société DUPONT DE NEMOURS ;
- des fibres à structure multicouche comprenant des couches alternées de polymères choisis parmi les polyesters, les polymères acryliques, les polyamides, telles que celles décrites dans les documents EP-A-6921217, EP-A-686858 et US-A-5472798. De telles fibres sont vendues sous les dénominations « Morphotex® », « Teijin Tetron Morphotex® » par la société TEIJIN.

Les fibres rigides particulièrement préférées sont les fibres de polymide-amide aromatiques.

Des fils ou fibres de polyimide-amide, qui peuvent être utilisés pour les compositions de l'invention, sont décrits, par exemple, dans le document de R. PIGEON et P. ALLARD, Chimie Macromoléculaire Appliquée, 40/41 (1974), pages 139-158 (n° 600), ou bien encore dans les documents US-A-3 802 841, FR-A-2 079 785, EP-A1-0 360 728, EP-A-0 549 494, auxquels on pourra se référer.

Les fibres de polyimide-amide aromatique préférées sont des fibres de polyimide-amide comprenant des motifs répétitifs de formule :

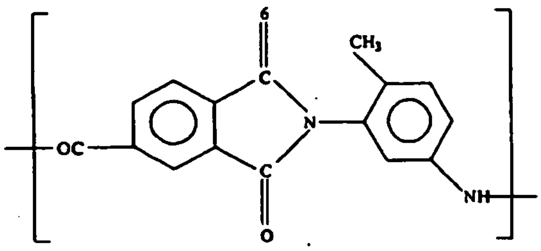

obtenus par polycondensation du toluylène diisocyanate et de l'anhydride triméllitique.

Les fibres peuvent êtres présentes dans la composition selon l'invention en une teneur allant de 0,01 % à 10 % en poids, par rapport au poids total de la composition, en particulier de 0,1 % à 5 % en poids, et plus particulièrement de 0,3 % à 3 % en poids.

Comme actifs cosmétiques pouvant être utilisés dans les compositions selon l'invention, on peut citer notamment des émollients, des hydratants, des vitamines et des filtres en particulier solaires.

Bien entendu, l'homme du métier veillera à choisir les éventuels additifs complémentaires et/ou leur quantité de telle manière que les propriétés avantageuses de la composition selon l'invention ne soient pas, ou substantiellement pas, altérées par l'adjonction envisagée.

## PROCEDE DE PREPARATION

Le procédé de préparation des compositions selon l'invention dépend en particulier de la nature de la ou des cires utilisées. Il dépend en particulier du fait que les cires soient de type traditionnel ou de type micro cire telles que définies précédemment. Pour les cires de type traditionnel, il dépend de plus du point de fusion commençante de ladite cire.

Les compositions de l'invention comprennent une ou plusieurs micro cires telles que définies précédemment.

De part sa formulation à l'état de particules, une telle cire peut être utilisée directement à une température inférieure à sa température de fusion. En d'autres termes dans une première variante de l'invention, on disperse directement les particules de micro cires dans la phase continue, et non pas en les y formant par l'intermédiaire d'étapes de fusion/recristallisation.

Cette étape de dispersion de la cire peut être en particulier effectuée à une température inférieure à la température de fusion de la cire et notamment à température ambiante, ce qui est bien entendu avantageux en terme de facilité de mise en oeuvre du procédé de préparation.

Le solvant non aqueux est choisi parmi ceux définis précédemment. Dans ce mode de réalisation particulier de procédé de préparation, l'eau et/ou le(s) solvant(s) hydrosoluble(s) et/ou les ingrédients supplémentaires tels que définis précédemment, peuvent être ajoutés selon les cas soit dans les produits de départ, soit dans la composition terminée.

Dans une deuxième variante de l'invention, le procédé de préparation des compositions met en oeuvre à la fois au moins une cire de type traditionnel et au moins une micro cire telles que définies précédemment. Dans un tel cas, on introduit généralement d'abord la cire de type traditionnel ou le mélange de cires de type traditionnel sous formes fondues au solvant non aqueux, le cas échéant en mélange avec au moins un polymère soluble dans ledit solvant et ayant une partie cristallisable, puis on agite ou malaxe le mélange ainsi obtenu pendant son refroidissement. La micro cire ou le mélange de micro cires n'est introduit que lorsque la température du mélange contenant la cire de type traditionnel est inférieure à la température de fusion de ladite micro cire ou inférieure à la température de fusion de la micro cire du mélange de micro cires ayant la température de fusion la plus basse, et notamment à température ambiante.

L'eau et/ou le(s) solvant(s) hydrosoluble(s) et les ingrédients supplémentaires peuvent être ajoutés selon les cas soit dans les produits de départ, soit dans la composition terminée, soit encore lorsque la composition est malaxée, au cours du refroidissement.

La présente invention a encore pour objet un procédé de maquillage des fibres kératiniques, dans lequel on applique sur lesdites fibres kératiniques notamment les cils, une composition telle que définie précédemment.

Les compositions de l'invention peuvent être en particulier appliquées sur les cils, à l'aide d'une brosse ou d'un peigne.

L'effet épaississant du maquillage, utilisant la composition de l'invention peut par ailleurs être renforcé en sélectionnant tout particulièrement le dispositif pour l'application de ladite composition.

En l'occurrence, il est particulièrement avantageux de procéder, dans le cas du maquillage des cils, à l'application de ladite composition avec une brosse de maquillage telle que décrite dans les brevets FR 2 701 198, FR 2 605 505, EP 792 603 et EP 663 161.

Les exemples qui suivent sont présentés à titre illustratif et non limitatif de l'invention.

## Protocole de préparation des compositions

*a. Procédé de préparation des composition ne comprenant que des cires sous forme de microparticules*

Dans au moins un solvant non aqueux, éventuellement en mélange avec au moins un polymère soluble dans ledit solvant non aqueux et ayant au moins une partie cristallisable, et dans un tel cas le mélange ayant été préalablement chauffé à une température de 45 °C puis refroidi jusqu'à température ambiante, on disperse sous agitation les matières colorantes ainsi que le gélifiant. Sous agitation, on ajoute alors la cire sous forme de microparticules, ainsi que le cas échéant, les ingrédients restants de la composition.

L'eau et/ou le(s) solvant(s) hydrosoluble(s) sont en particulier dispersés progressivement sous agitation.

*b. Préparation des compositions comprenant à la fois des cires de type traditionnel et des cires sous forme de microparticules*

Dans au moins un solvant non aqueux, éventuellement en mélange avec au moins un polymère soluble dans ledit solvant non aqueux et ayant au moins une partie cristallisable, chauffé à une température de 45 °C puis refroidi jusqu'à température ambiante, on disperse sous agitation les matières colorantes ainsi que le gélifiant. Le mélange obtenu est ensuite chauffé à 45 °C puis le mélange des cires traditionnelles préalablement chauffées jusqu'à fusion complète est ajouté progressivement. On laisse le mélange ainsi obtenu revenir à température ambiante sous agitation. On ajoute alors la cire sous forme de microparticules, ainsi que le cas échéant, les ingrédients restants de la composition.

L'eau et/ou le(s) solvant(s) hydrosoluble(s) sont en particulier dispersés progressivement sous agitation.

*c. Préparation des compositions à l'aide d'un malaxeur bi-vis en continu*

La préparation s'effectue dans un malaxeur bi-vis en continu tel que le modèle « BC-21 » de la société CLEXTRAL et se fait dans les conditions suivantes :

- température d'entrée : 100 °C
- température de sortie : 20 °C
- débit = 3 kg/h
- vitesse des vis : 600 tours/min.

Les cires préalablement fondues sont introduites en tête du malaxeur en même temps que le solvant non aqueux et les autres ingrédients puis le mélange est refroidi sous malaxage bi-vis en continu jusqu'à la température de sortie.

## Mesures des caractéristiques Physiques

La mesure de la teneur en matière sèche est réalisée selon le protocole décrit précédemment.

Les mesures de rhéologie ont été effectuées selon les protocoles décrits précédemment en utilisant un rhéomètre à contrainte imposée « Haake RheoStress 600® » dans les conditions suivantes :

- température de mesure 25 °C,
- palier de 180 sec à 25 °C avant le début de la mesure,
- balayage en contrainte de 1 à 10 000 Pa,
- fréquence de mesure : 1 Hz.

## Exemples 1, 2 et 3

On a préparé trois mascaras waterproof selon le procédé décrit en b) ayant respectivement les compositions présentées dans le tableau 1 ci-dessous (dans ce tableau, les quantités indiquées sont en pourcentage pondéral et exprimées par rapport au poids total de la composition) :

TABLEAU I

| | Exemple 1 | Exemple 2 | Exemple 3 |
|---|---|---|---|
| Cire d'abeille | 8,67 | 8,67 | 8,67 |
| Micro cire de carnauba (« MicroCare 350® » de MICRO POWDERS) | 24,2 | 24,2 | 24,2 |
| Micro cire synthétique (« MicroEase 114S® » de MICRO POWDERS) | 2,02 | 2,02 | 2,02 |
| Polylaurate de vinyle (« Mexomère PP® » de CHIMEX) | 0,66 | 0,66 | 0,66 |
| Conservateur | 0,2 | 0,2 | 0,2 |
| Colorant | 5,7 | 5,7 | 5,7 |
| Bentonite | 3,6 | 3,6 | 3,6 |
| Carbonate de propylène | 1,18 | 1,18 | 1,18 |
| Copolymère stéarate d'allyle / acétate de vinyle (« Mexomère PQ® » de CHIMEX) | 2 | *** | *** |
| Copolymère éthylène diamine / dilinoléate de stéaryle (« Uniclear 100 VG® » d'ARIZONA CHEMICAL) | *** | 2 | *** |
| Polymère d'acrylate d'alkyle en $C_{10}$-$C_{30}$ (de LANDEC) | *** | *** | 2 |
| Isododécane | 51,8 | 51,8 | 51,8 |

On a étudié différentes caractéristiques *in vitro* de ces compositions selon les protocoles décrits précédemment.
Les résultats sont présentés dans le tableau II suivant :

| Caractéristiques / Compositions | [Cire] (% m) | ES (% m) | Gp (Pa) | $\tau_c$ (Pa) |
|---|---|---|---|---|
| Exemple 1 | 34,9 | 48,5 | 4500 | 40 |
| Exemple 2 | 34,9 | 48,2 | 2300 | 30 |
| Exemple 3 | 34,9 | 48,5 | 20 000 | 100 |

TABLEAU II

Les compositions obtenues présentent donc des teneurs en matière sèche particulièrement élevées associées à un module de rigidité plateau suffisamment faible pour permettre leur utilisation dans des conditions satisfaisantes.

## Exemple 4

On a préparé un mascara waterproof selon le procédé b) ayant la composition suivante :

- Cire d'abeille 8,67 g
- Micro cire de Carnauba (« MicroCare 350® » de MICRO POWDERS) 24,20 g
- Micro cire synthétique (« MicroEase 114® » de MICRO POWDERS) 2,02 g
- Polylaurate de vinyle (« Mexomère PP® » de CHIMEX) 0,66 g
- Conservateur 0,20 g
- Colorant 5,70 g
- Bentonite 3,60 g
- Carbonate de propylène 1,18 g
- Copolymère stéarate d'allyle / acétate de vinyle (« Mexomère PQ® » de CHIMEX) 6,50 g

- Isododécane 47,27 g

On a étudié différentes caractéristiques *in vitro* de cette composition selon les protocoles décrits précédemment. Les résultats sont présentés dans le tableau III ci-dessous :

| Caractéristiques / Composition | [Cire] (% m) | ES (% m) | Gp (Pa) | $\tau_c$ (Pa) |
|---|---|---|---|---|
| Exemple 4 | 34,9 | 53,0 | 1 000 | 9 |

TABLEAU III

La composition obtenue présente donc une teneur en matière sèche très élevée (supérieure à 50 %) avec un module de rigidité particulièrement faible (1 000 Pa).

**Exemples 5 et 6**

On a préparé selon le procédé a), deux mascaras waterproof ayant respectivement les compositions présentées dans le tableau IV ci-dessous (dans ce tableau, les quantités indiquées sont en pourcentage pondéral et exprimées par rapport au poids total de la composition) :

TABLEAU IV

| | Exemple 5 | Exemple 6 |
|---|---|---|
| Micro cire de carnauba (« MicroCare 350® » de MICRO POWDERS) | 45 | *** |
| Micro cire synthétique (« MicroEase 114S® » de MICRO POWDERS) | *** | 45 |
| Colorant | 6 | 6 |
| Bentonite | 3,6 | 3,6 |
| Carbonate de propylène | 1,18 | 1,18 |
| Copolymère stéarate d'allyle / acétate de vinyle (« Mexomère PQ® » de CHIMEX) | 6 | 6 |
| Isododécane | 38,22 | 38,22 |

On a étudié les différentes caractéristiques de ces compositions *in vitro* selon les protocoles décrits précédemment.
Les résultats sont présentés dans le tableau V ci-dessous :

| Caractéristiques / Compositions | [Cire] (% m) | ES (% m) | Gp (Pa) | τc (Pa) |
|---|---|---|---|---|
| Exemple 5 | 45 | 61,8 | 3 000 | 40 |
| Exemple 6 | 45 | 61,8 | 160 | 25 |

TABLEAU V

On constate que les compositions obtenues présentent des teneurs en matière sèche supérieures à 60 % tout en présentant des modules de rigidité faibles et donc une souplesse satisfaisante.
Ces formulations s'appliquent particulièrement bien sur les cils et conduisent à un maquillage donnant un épaississement des cils très important.

**Exemples 7 à 10**

On a préparé quatre mascaras waterproof selon le procédé décrit en b) par mélange des ingrédients présentés dans le tableau VI ci-dessous (dans ce tableau, les quantités indiquées sont en pourcentage pondéral et exprimées par rapport au poids total de la composition).

TABLEAU VI

| | Exemple 7 | Exemple 8 | Exemple 9 | Exemple 10 |
|---|---|---|---|---|
| Cire d'abeille | 8,67 | 8,67 | 13 | 13 |
| Micro Cire de carnauba (« MicroCare 350® » de MICRO POWDERS) | 12,2 | 16,2 | 18,37 | 18,37 |
| Micro Cire synthétique (« MicroEase 114S® » de MICRO POWDERS) | 6,52 | 6,52 | 6,52 | 6,52 |
| Polytaurate de vinyle (« Mexomère PP® » de CHIMEX) | 0,66 | 0,66 | *** | *** |
| Conservateur | 0,2 | 0,2 | 0,2 | 0,2 |
| Colorant | 5,7 | 5,7 | 5,7 | 5,7 |
| Hectorite modifiée (« Bentone 38V® » d'ELEMENTIS) | 3,6 | 3,6 | 3,6 | 3,6 |
| Carbonate de propylène | 1,18 | 1,18 | 1,18 | 1,18 |
| Copolymère vinyle pyrrolidone/1-Eicosène (« Antaron V 220® » d'ISP) | *** | 5 | *** | *** |
| Copolymère stéarate d'allyle/acétate de vinyle (« Mexomère PQ® » de CHIMEX) | 6,5 | 6,5 | 6,5 | 6,5 |
| Copolymère éthylènediamine/dilinoléate de stéaryle (« Uniclear 1.00 VG® » d'ARIZONA CHEMICAL) | 3 | 3 | 1,5 | 1,5 |
| Copolymère acétate de vinyle/t-butyl benzoate de vinyle/ acide crotonique en dispersion aqueuse à 26,3 % en MA) (« Mexomère PAM® » de CHIMEX) | 3 | 3 | *** | *** |
| Copolymère acrylate d'éthyle/méthacrylate de méthyle (80/20) en dispersion aqueuse à 50 % en MA (« Daitosol 5000 AD® » de DAITO) | 9 | *** | *** | *** |
| Eau | *** | *** | 7,32 | *** |
| Isododécane | 39,77 | 39,77 | 36,11 | 43,43 |

Les compositions des exemples 7 et 8 comprennent en fait de l'eau en des proportions respectives de 6,5 et 2,1 % en poids par rapport au poids total de la composition, l'eau provenant des latex utilisés, à savoir le « Mexomère PAM® » de la société CHIMEX et le « Daitosol 5000 AD® » de la société DAITO.

On a étudié différentes caractéristiques *in vitro* de ces compositions selon les protocoles décrits précédemment.

Les résultats sont présentés dans le tableau VII ci-dessous.

| Caractéristiques / Compositions | [cire] (% m) | ES (%m) | Gp (Pa) | τc (Pa) |
|---|---|---|---|---|
| Exemple 7 | 27,4 | 53,0 | 6 000 | 53,7 |
| Exemple 8 | 27,4 | 57,5 | 7 000 | 60 |
| Exemple 9 | 37,9 | 56,5 | 13 000 | 70 |
| Exemple 10 | 37,9 | 56,8 | 2 800 | 20 |

TABLEAU VII

## Exemple 12

On a préparé selon le procédé décrit en b) un mascara waterproof ayant la composition suivante :

- Cire microcristalline (« Microwax HW® » de PARAMELT) 30 g
- Pigments 9,24 g
- Amidon de riz 1,68 g
- Copolymère acétate de vinyle/stéarate d'allyle (« Mexomère PQ® » de CHIMEX) 4,42 g
- Polylaurate de vinyle (« Mexomère PP® » de CHIMEX) 1,5 g
- Hectorite modifiée (« Bentone 38V® » d'ELEMENTIS) 0,63 g
- Carbonate de propylène 0,21 g
- Alcool éthylique 3,0 g
- Stéarate de l'oligomère d'acide 12-hydroxystéarique (« Solsperse 21000® » d'AVECIA) 0,2 g
- Conservateur 0,4 g
- Isododécane 48,72 g

Cette composition présente une teneur en matière sèche de 48,28 % en poids par rapport au poids total de la composition.

## Revendications

1. Composition cosmétique de maquillage de fibres kératiniques **caractérisée en ce qu'**elle comprend un milieu solvant non aqueux, **caractérisée en ce que** ledit milieux solvant non aqueux comprend au moins un composé volatil, non soluble dans l'eau, et liquide à température ambiante, **en ce qu'**elle comprend en outre au moins un polymère soluble dans ledit milieu solvant non aqueux et ayant au moins une partie cristallisable, **en ce qu'**elle comprend au plus 20 % en poids d'eau et/ou de solvant hydrosoluble par rapport au poids total de la composition, **en ce qu'**elle possède une teneur en matière sèche supérieure à 45 % en poids par rapport au poids total de la composition et un module de rigidité plateau Gp inférieur ou égal à 28 000 Pa, et **en ce qu'**elle comprend au moins une micro cire ayant une dimension de 0,5 à 30 micromètres, et **en ce que** la teneur totale en cire est supérieure à 25 % en poids par rapport au poids total de la composition,
   la mesure du module de rigidité plateau Gp étant mesurée selon la méthode définie dans la description.

2. Composition selon la revendication 1, **caractérisée en ce que** ladite teneur en matière sèche est de 46 à 80 %, en particulier de 48 à 70 % et plus particulièrement de 50 à 65 % en poids par rapport au poids total de la composition.

3. Composition selon la revendication. 1 ou 2, **caractérisée en ce que** ledit module de rigidité plateau Gp est inférieur ou égal à 25 000 Pa, voire à 20 000 Pa.

4. Composition selon l'une quelconque des revendications précédentes,
   **caractérisée en ce que**, ledit composé volatil représente plus de 50 % en poids dudit milieu solvant non aqueux.

5. Composition selon l'une quelconque des revendications précédentes,
   **caractérisée en ce que** ledit composé volatil, non soluble dans l'eau, est choisi parmi les huiles hydrocarbonées,

siliconées et/ou fluorées, les solvants organiques et leurs mélanges, et en particulier parmi les huiles hydrocarbonées ayant de 8 à 16 atomes de carbone et leurs mélanges.

6. Composition selon l'une quelconque des revendications précédentes, **caractérisée en ce que** ledit composé volatil, non soluble dans l'eau, est présent dans la composition en une teneur inférieure à 55 %, notamment comprise entre 10 et 54 %, en particulier entre 15 et 52 %, et plus particulièrement entre 17,5 et 50 % en poids par rapport au poids total de la composition.

7. Composition selon l'une quelconque des revendications précédentes, **caractérisée en ce que** ledit milieu solvant non aqueux comprend en outre au moins une huile non volatile.

8. Composition selon l'une quelconque des revendications précédentes, **caractérisée en ce que** ladite composition est exempte d'eau et de solvant hydrosoluble.

9. Composition selon l'une quelconque des revendications 1 à 7, **caractérisée en ce que** la teneur totale en eau et/ou en solvant(s) hydrosoluble(s) est supérieure ou égale à 0,5 %, en particulier de 1 à 18 %, et plus particulièrement de 2 à 15 % en poids par rapport au poids total de la composition.

10. Composition selon la revendication précédente, **caractérisée en ce que** ledit solvant hydrosoluble est choisi parmi les monoalcools inférieurs ayant de 1 à 5 atomes de carbone, les glycols ayant de 2 à 8 atomes de carbone, les cétones en $C_3$ et $C_4$ et les aldéhydes en $C_2$ à $C_4$.

11. Composition selon l'une quelconque des revendications précédentes, **caractérisée en ce que** ladite cire est choisie parmi les cires, solides et rigides à température ambiante ayant un point de fusion supérieur ou égal à 30 °C, en particulier supérieur ou égal à 45 °C, notamment supérieur ou égal à 55 °C.

12. Composition selon l'une quelconque des revendications précédentes, **caractérisée en ce que** la cire est choisie parmi les cires hydrocarbonées comme la cire d'abeille, la cire de lanoline, les cires d'insectes de Chine, la cire de sumac, les paraffines, les cires de polyéthylène, les copolymères cireux ainsi que leurs esters ; les cires obtenues par hydrogénation catalytique d'huiles animales ou végétales ayant des chaînes grasses, linéaires ou ramifiées en $C_8$-$C_{32}$ comme l'huile de jojoba partiellement hydrogénée isomérisée trans, l'huile de tournesol hydrogénée, l'huile de ricin hydrogénée, l'huile de coprah hydrogénée, l'huile de lanoline hydrogénée et le tétrastéarate de di-(triméthylol-1,1,1 propane) et les cires obtenues par hydrogénation d'huile de ricin estérifiée avec l'alcool cétylique.

13. Composition selon l'une quelconque des revendications 1 à 11, **caractérisée en ce que** la cire est choisie parmi les cires possédant un collant supérieur ou égal à 0,7 N.s, en particulier supérieur ou égal à 1 N.s, et une dureté inférieure ou égale à 3,5 MPa.

14. Composition selon la revendication précédente, **caractérisée en ce que** ladite cire est choisie parmi les (hydroxystéaryloxy) stéarate d'alkyle en $C_{20}$-$C_{40}$.

15. Composition selon l'une quelconque des revendications 1 à 12, **caractérisée en ce que** ladite cire est choisie parmi les cires ayant un point de fusion commençante supérieur ou égal à 45 °C, notamment supérieur ou égal à 50 °C, en particulier supérieur ou égal à 55 °C et plus particulièrement supérieur ou égal à 60 °C.

16. Composition selon la revendication précédente, **caractérisée en ce que** ladite cire est choisie parmi la cire de carnauba, la cire de son de riz, la cire de Candellila, la cire d'Ouricurry, la cire de montan, l'ozokérite, les cires obtenues par la synthèse de Fisher-Tropsch, l'huile de jojoba hydrogénée, le tétrabéhénate de di-(triméthylol-1,1,1 propane), les cires obtenues par hydrogénation catalytique d'huile d'olive estérifiée avec l'alcool stéarylique, les cires microcristallines et des cires de polyéthylène.

17. Composition selon l'une quelconque des revendications précédentes, **caractérisée en ce que** la teneur totale en cire est de 25 à 70 % notamment de 25 à 65 % et en particulier de 25 à 60 %, voire de 25 à 55 % en poids par rapport au poids total de la composition.

18. Composition selon l'une quelconque des revendications précédentes, **caractérisée en ce que** ledit polymère soluble dans ledit milieu solvant non aqueux et ayant au moins une partie cristallisable a une masse molaire allant de 200 à 1000 000 g/mol, en particulier de 500 à 500 000 g/mol et plus

particulièrement de 1000 à 300 000 g/mol.

**19.** Composition selon l'une quelconque des revendications précédentes, **caractérisée en ce que** la partie cristallisable du polymère soluble dans ledit milieu solvant non aqueux et ayant au moins une partie cristallisable représente au moins 5 %, en particulier au moins 10 % et au plus 50 %, et plus particulièrement de 30 à 50 % en poids du poids total dudit polymères.

**20.** Composition selon l'une quelconque des revendications précédentes, **caractérisée en ce que** ledit polymère soluble dans ledit milieu solvant non aqueux et ayant au moins une partie cristallisable est choisi parmi les copolymères de (méth)acrylates d'alkyles linéaires et saturés en $C_{12}$ à $C_{30}$ et de (méth)acrylates d'alkyles en $C_4$ à $C_{10}$ linéaires ou en $C_4$ à $C_{30}$ ramifiés ou cycliques et/ou insaturés, les copolymères d'esters vinyliques à groupes alkyles linéaires et saturés en $C_{12}$ à Z30 et d'esters vinyliques à groupes alkyles en $C_4$ à $C_{10}$ linéaires, ou en $C_4$ à $C_{30}$ ramifiés ou cycliques et/ou insaturés, les polycondensats de type polyamide résultant de la condensation entre ($\alpha$) au moins un acide choisi parmi les acides dicarboxyliques comprenant au moins 32 atomes de carbone et ($\beta$) un alkylène diamine, ledit polycondensat comprenant au moins un groupe acide carboxylique terminal estérifié ou amidifié avec au moins un mono alcool ou un mono amine comprenant de 12 à 30 atomes de carbone linéaires et saturés et les polycondensats polyesters lipophiles dont les extrémités sont estérifiées par un acide ou un alcool cristallisable constitué d'une chaîne carbonée linéaire saturée en $C_{12}$ à $C_{30}$.

**21.** Composition selon l'une quelconque des revendications précédentes, **caractérisée en ce que** ledit polymère soluble dans ledit milieu solvant non aqueux et ayant au moins une partie cristallisable est choisi parmi les copolymères acétate de vinyle/stéarate de vinyle, acétate de vinyle/stéarate d'allyle, acétate de vinyle/éthylène, éthylène diamine/dilinoléate de stéaryle, les copolymères séquencés butadiène hydrogéné/isoprène et l'acide 12- polyhydroxystéarique estérifié à l'une de ses extrémités par l'acide stéarique.

**22.** Composition selon l'une quelconque des revendications précédentes, **caractérisée en ce que** ledit polymère soluble dans ledit milieu solvant non aqueux et ayant au moins une partie cristallisable est présent en une teneur allant de 0,01 à 30 %, notamment de 0,1 à 20 %, et en particulier de 1 à 10 % en poids par rapport au poids total de la composition.

**23.** Composition selon l'une quelconque des revendications précédentes, **caractérisée en ce qu'**elle comprend en outre au moins un polymère filmogène.

**24.** Composition selon l'une quelconque des revendications précédentes, **caractérisée en ce qu'**elle comprend en outre au moins une matière colorante.

**25.** Composition selon l'une quelconque des revendications précédentes, **caractérisée en ce qu'**elle comprend en outre au moins une charge.

**26.** Composition selon l'une quelconque des revendications précédentes,
**caractérisée en ce qu'**elle comprend en outre au moins un additif cosmétiquement acceptable choisi parmi les antioxydants, les conservateurs, les parfums, les neutralisants, les plastifiants, les fibres, les gélifiants, les actifs cosmétiques et leurs mélanges.

**27.** Composition selon l'une quelconque des revendications précédentes, **caractérisée en ce qu'**elle possède un seuil d'écoulement $\tau_c$ mesuré par rhéologie oscillatoire $\gamma$ =1 Hz) variant de 10 à 200 Pa et notamment de 20 à 100 Pa.

**28.** Procédé de préparation d'une composition telle que définie selon l'une quelconque des revendications 1 à 27, **caractérisé en ce qu'**il comprend au moins une étape de dispersion d'au moins une cire à l'état de particules ayant une dimension de 0,5 $\mu$m à 30 $\mu$m, dans au moins un solvant non aqueux, ledit solvant étant à une température inférieure à celle de fusion de ladite cire à l'état de particules.

**29.** Procédé selon la revendication précédente; **caractérisé en ce que** la dispersion est effectuée à température ambiante.

**30.** Procédé selon la revendication 28 ou 29, **caractérisé en ce que** ladite dimension est de 1 à 20 $\mu$m, et en particulier de 5 à 10 $\mu$m.

**31.** Procédé selon l'une quelconque des revendications 28 à 30, **caractérisé en ce que** ladite cire est choisie parmi la cire de carnauba, la cire synthétique, les cires constituées d'un mélange de cire de carnauba et de cire de polyéthylène, les cires constituées d'un mélange de cire de carnauba et de cire synthétique, les cires de polyéthylène et les cires de polytétrafluoroéthylène.

**32.** Procédé selon l'une quelconque des revendications 28 à 31, **caractérisé en ce que** l'on introduit préalablement au moins une cire telle que définie selon l'une quelconque des revendications 11 à 16 sous forme fondue dans ledit solvant, puis l'on laisse le mélange ainsi obtenu refroidir sous agitation ou malaxage jusqu'à une température au moins inférieure à celle de fusion de ladite cire à l'état de particules.

**33.** Procédé selon l'une quelconque des revendications 28 à 32, **caractérisé en ce que** ledit solvant non aqueux est tel que défini dans l'une quelconque des revendications 1 ou 5à7.

**34.** Procédé de maquillage des fibres kératiniques, **caractérisé en ce que** l'on' applique sur lesdites fibres kératiniques notamment les cils, une composition telle que définie dans l'une quelconque des revendications 1 à 27 ou telle qu'obtenue par un procédé tel que défini dans l'une quelconque des revendications 28 à 33.

## Claims

**1.** Cosmetic composition for making up keratin fibres, **characterized in that** it comprises a nonaqueous solvent medium, **characterized in that** said nonaqueous solvent medium comprises at least one water-insoluble volatile compound that is liquid at room temperature, and **in that** it comprises at least one polymer that is soluble in said nonaqueous solvent medium and that has at least one crystallizable portion, and **in that** it comprises up to 20% by weight of water and/or of water-soluble solvent relative to the total weight of the composition, **in that** it has a solids content of greater than 45% by weight relative to the total weight of the composition and a plateau modulus of stiffness Gp of less than or equal to 28 000 Pa, and **in that** it comprises at least one microwax with a size of 0.5 to 30 micrometres, and **in that** the total wax(es) content is greater than 25% by weight relative to the total weight of the composition, the plateau modulus of stiffness Gp being measured according to the method defined in the description.

**2.** Composition according to Claim 1, **characterized in that** said solids content is from 46% to 80%, in particular from 48% to 70% and more particularly from 50% to 65% by weight, relative to the total weight of the composition.

**3.** Composition according to Claim 1 or 2,
**characterized in that** said plateau modulus of stiffness Gp is less than or equal to 25 000 Pa or even 20 000 Pa.

**4.** Composition according to any one of the preceding claims, **characterized in that** said volatile compound represents more than 50% by weight of said nonaqueous solvent medium.

**5.** Composition according to any one of the preceding claims, **characterized in that** said water-insoluble volatile compound is chosen from hydrocarbon-based oils, silicone oils and/or fluoro oils, and organic solvents, and mixtures thereof, and in particular from hydrocarbon-based oils containing from 8 to 16 carbon atoms, and mixtures thereof.

**6.** Composition according to any one of the preceding claims, **characterized in that** said water-insoluble volatile compound is present in the composition in a content of less than 55%, especially between 10% and 54%, in particular between 15% and 52% and more particularly between 17.5 and 50% by weight relative to the total weight of the composition.

**7.** Composition according to any one of the preceding claims, **characterized in that** the nonaqueous solvent medium also comprises at least one nonvolatile oil.

**8.** Composition according to any one of the preceding claims, **characterized in that** said composition is free of water and of water-soluble solvent.

**9.** Composition according to any one of Claims 1 to 7, **characterized in that** the total content of water and/or of water-soluble solvent(s) is greater than or equal to 0.5%, in particular from 1% to 18% and more particularly from 2% to 15% by weight relative to the total weight of the composition.

**10.** Composition according to the preceding claim, **characterized in that** said water-soluble solvent is chosen from lower monoalcohols containing from 1 to 5 carbon atoms, glycols containing from 2 to 8 carbon atoms, $C_3$ and $C_4$ ketones and $C_2$ to $C_4$ aldehydes.

**11.** Composition according to any one of the preceding claims, **characterized in that** said wax is chosen from waxes that are solid and rigid at room temperature, with a melting point of greater than or equal to 30°C, in particular greater than or equal to 45°C and especially greater than or equal to 55°C.

**12.** Composition according to any one of the preceding claims, **characterized in that** the wax is chosen from hydrocarbon-based waxes, for instance beeswax, lanolin wax, Chinese insect waxes, sumach wax, paraffins, polyethylene waxes, waxy copolymers, and esters thereof; the waxes obtained by catalytic hydrogenation of animal or plant oils containing linear or branched $C_8$-$C_{32}$ fatty chains, for instance trans-isomerized partially hydrogenated jojoba oil, hydrogenated sunflower oil, hydrogenated castor oil, hydrogenated coconut oil, hydrogenated lanolin oil and bis (1,1,1-trimethylolpropane) tetrastearate and the waxes obtained by hydrogenation of castor oil esterified with cetyl alcohol.

**13.** Composition according to any one of Claims 1 to 11, **characterized in that** the wax is chosen from waxes with a tack of greater than or equal to 0.7 N.s and in particular greater than or equal to 1 N.s, and a hardness of less than or equal to 3.5 MPa.

**14.** Composition according to the preceding claim, **characterized in that** said wax is chosen from $C_{20}$-$C_{40}$ alkyl (hydroxystearyloxy) stearates.

**15.** Composition according to any one of Claims 1 to 12, **characterized in that** said wax is chosen from waxes with a starting melting point of greater than or equal to 45°C, especially greater than or equal to 50°C, in particular greater than or equal to 55°C and more particularly greater than or equal to 60°C.

**16.** Composition according to the preceding claim, **characterized in that** said wax is chosen from carnauba wax, rice bran wax, candelilla wax, ouricurry wax, montan wax, ozokerites, the waxes obtained by Fisher-Tropsch synthesis, hydrogenated jojoba oil, bis(1,1,1-trimethylolpropane) tetrabehenate, the waxes obtained by catalytic hydrogenation of olive oil esterified with stearyl alcohol, microcrystalline waxes and polyethylene waxes.

**17.** Composition according to any one of the preceding claims, **characterized in that** the total wax content is from 25% to 70%, especially from 25% to 65%, and in particular from 25% to 60%, indeed from 25% to 55% by weight relative to the total weight of the composition.

**18.** Composition according to any one of the preceding claims, **characterized in that** said polymer that is soluble in said nonaqueous solvent medium and that has at least one crystallizable portion, has a molar mass ranging from 200 to 1 000 000 g/mol, in particular from 500 to 500 000 g/mol and more particularly from 1000 to 300 000 g/mol.

**19.** Composition according to any one of the preceding claims, **characterized in that** the crystallizable portion of the polymer that is soluble in said nonaqueous solvent medium and that has at least one crystallizable portion, represents at least 5%, in particular at least 10% and not more than 50%, and more particularly from 30% to 50%, by weight relative to the total weight of said polymer.

**20.** Composition according to any one of the preceding claims, **characterized in that** said polymer that is soluble in said nonaqueous solvent medium and that has at least one crystallizable portion, is chosen from copolymers of linear and saturated $C_{12}$ to $C_{30}$ alkyl (meth)acrylates and of linear $C_4$ to $C_{10}$ or branched, cyclic and/or unsaturated $C_4$ to $C_{30}$ alkyl (meth)acrylates, copolymers of vinyl esters containing linear and saturated $C_{12}$ to $C_{30}$ alkyl groups and of vinyl esters containing linear $C_4$ to $C_{10}$ or branched, cyclic and/or unsaturated $C_4$ to $C_{30}$ alkyl groups, polycondensates of polyamide type resulting from the condensation between ($\alpha$) at least one acid chosen from dicarboxylic acids containing at least 32 carbon atoms and ($\beta$) an alkylenediamine, said polycondensate comprising at least one carboxylic acid end group esterified or amidated with at least one linear and saturated monoalcohol or one linear and saturated monoamine containing from 12 to 30 carbon atoms, and lipophilic polyester polycondensates whose ends are esterified with a crystallizable acid or alcohol consisting of a saturated linear $C_{12}$ to $C_{30}$ carbon-based chain.

**21.** Composition according to any one of the preceding claims, **characterized in that** said polymer that is soluble in

said nonaqueous solvent medium and that has at least one crystallizable portion, is chosen from vinyl acetate/vinyl stearate, vinyl acetate/allyl stearate, vinyl acetate/ethylene and ethylenediamine/stearyl dilinoleate copolymers, block copolymers of hydrogenated butadiene/isoprene and poly(12-hydroxystearic acid) which is esterified at one of its ends with stearic acid.

**22.** Composition according to any one of the preceding claims, **characterized in that** said polymer that is soluble in said nonaqueous solvent medium and that has at least one crystallizable portion, is present in a content ranging from 0.01% to 30%, especially from 0.1% to 20% and in particular from 1% to 10% by weight relative to the total weight of the composition.

**23.** Composition according to any one of the preceding claims, **characterized in that** it also comprises at least one film-forming polymer.

**24.** Composition according to any one of the preceding claims, **characterized in that** it also comprises at least one dyestuff.

**25.** Composition according to any one of the preceding claims, **characterized in that** it also comprises at least one filler.

**26.** Composition according to any one of the preceding claims, **characterized in that** it also comprises at least one cosmetically acceptable additive chosen from antioxidants, preserving agents, fragrances, neutralizers, plasticizers, fibres, gelling agents and cosmetic active agents, and mixtures thereof.

**27.** Composition according to any one of the preceding claims, **characterized in that** it has a flow threshold $\tau_c$, measured by oscillating rheology ($\gamma$ = 1 Hz), ranging from 10 to 200 Pa and especially from 20 to 100 Pa.

**28.** Process for preparing a composition as defined according to any one of Claims 1 to 27, **characterized in that** it comprises at least one step of dispersing at least one wax in the form of particles between 0.5 $\mu$m and 30 $\mu$m in diameter in at least one nonaqueous solvent, said solvent being at a temperature below the melting point of said wax in particle form.

**29.** Process according to the preceding claim, **characterized in that** the dispersion is performed at room temperature.

**30.** Process according to Claim 28 or 29, **characterized in that** said size is from 1 to 20 $\mu$m and in particular from 5 to 10 $\mu$m.

**31.** Process according to any one of Claims 28 to 30, **characterized in that** said wax is chosen from carnauba wax, synthetic wax, waxes consisting of a mixture of carnauba wax and of polyethylene wax, waxes consisting of a mixture of carnauba wax and synthetic wax, polyethylene waxes and polytetrafluoroethylene waxes.

**32.** Process according to any one of Claims 28 to 31, **characterized in that** at least one wax as defined according to any one of Claims 11 to 16 is introduced beforehand in molten form in said solvent, and the mixture thus obtained is then allowed to cool with stirring or is blended until it is at a temperature at least below the melting point of said wax in particle form.

**33.** Process according to any one of Claims 28 to 32, **characterized in that** said nonaqueous solvent is as defined in any one of Claims 1 or 5 to 7.

**34.** Process for making up keratin fibres, **characterized in that** a composition as defined in any one of Claims 1 to 27 or as obtained via a process as defined in any one of Claims 28 to 33 is applied to said keratin fibres, especially the eyelashes.

### Patentansprüche

**1.** Kosmetische Zusammensetzung zum Schminken von Keratinfasern, **dadurch gekennzeichnet, dass** sie ein nicht wässriges Lösungsmittel umfasst, **dadurch gekennzeichnet, dass** das nicht wässrige Lösungsmittel mindestens eine im Wasser nicht lösliche und bei Umgebungstemperatur flüssige flüchtige Verbindung umfasst, dass sie außerdem mindestens ein Polymer umfasst, das in dem nicht wässrigen Lösungsmittelmedium löslich ist und mindestens einen kristallisierbaren Teil aufweist, dass sie höchstens 20 Gew.-% Wasser und/oder wasserlösliches Lösungsmittel, bezogen auf das Gesamtgewicht der Zusammensetzung, umfasst, dass sie einen Gehalt an Trockenmasse von mehr als 45 Gew.-%, bezogen auf das Gesamtgewicht der Zusammensetzung, und einen Starrheits-Plateaumodul Gp kleiner oder gleich 28 000 Pa besitzt und dass sie mindestens ein Mikrowachs mit einer Größe von 0,5 bis 30 Mikrometer umfasst und dass der Gesamtgehalt an Wachs(e) größer als 25 Gew.-%, bezogen auf das Gesamtgewicht der Zusammensetzung ist,
wobei die Messung des Starrheits-Plateaumoduls Gp nach der in der Beschreibung definierten Methode gemessen wird.

**2.** Zusammensetzung nach Anspruch 1, **dadurch gekennzeichnet, dass** der Trockenmassegehalt 46 bis 80%, insbesondere 48 bis 70% und ganz besonders 50 bis 65 Gew.-%, bezogen auf das Gesamtgewicht der Zusammensetzung, beträgt.

**3.** Zusammensetzung nach Anspruch 1 oder 2, **dadurch gekennzeichnet, dass** der Starrheits-Plateaumodul Gp kleiner oder gleich 25 000 Pa oder sogar kleiner oder gleich 20 000 Pa ist.

**4.** Zusammensetzung nach einem der vorhergehenden Ansprüche, **dadurch gekennzeichnet, dass** die flüchtige Verbindung mehr als 50 Gew.-% des nicht wässrigen Lösungsmittelmediums darstellt.

**5.** Zusammensetzung nach einem der vorhergehenden Ansprüche, **dadurch gekennzeichnet, dass** die in Wasser nicht lösliche flüchtige Verbindung aus den siliconierten und/oder fluorierten Kohlenwasserstoff ölen, den organischen Lösungsmitteln und ihren Mischungen und insbesondere aus den Kohlenwasserstoffölen mit 8 bis 16 Kohlenstoffatomen und ihren Mischungen ausgewählt ist.

**6.** Zusammensetzung nach einem der vorhergehenden Ansprüche, **dadurch gekennzeichnet, dass** die in Wasser nicht lösliche flüchtige Verbindung in der Zusammensetzung in einem Gehalt von weniger als 55 %, vor allem zwischen 10 und 54 %, insbesondere zwischen 15 und 52 % und ganz besonders zwischen 17,5 und 50 Gew.-%, bezogen auf das Gesamtgewicht der Zusammensetzung, vorliegt.

**7.** Zusammensetzung nach einem der vorhergehenden Ansprüche, **dadurch gekennzeichnet, dass** das nicht wässrige Lösungsmittelmedium außerdem mindestens ein nicht flüchtiges Öl umfasst.

**8.** Zusammensetzung nach einem der vorhergehenden Ansprüche, **dadurch gekennzeichnet, dass** die Zusammensetzung frei von Wasser und wasserlöslichem Lösungsmittel ist.

**9.** Zusammensetzung nach einem der Ansprüche 1 bis 7, **dadurch gekennzeichnet, dass** der Gesamtgehalt an Wasser und/oder an wasserlöslichem bzw. wasserlöslichen Lösungsmitteln größer oder gleich 0,5 %, insbesondere 1 bis 18 % und ganz besonders 2 bis 15 Gew.-%, bezogen auf das Gesamtgewicht der Zusammensetzung, ist.

**10.** Zusammensetzung nach dem vorhergehenden Anspruch, **dadurch gekennzeichnet, dass** das wasserlösliche Lösungsmittel aus den niedrigen Monoalkoholen mit 1 bis 5 Kohlenstoffatomen, den Glykolen mit 2 bis 8 Kohlenstoffatomen, den $C_3$-$C_4$-Ketonen und den $C_2$-$C_4$-Aldehyden ausgewählt ist.

**11.** Zusammensetzung nach einem der vorhergehenden Ansprüche, **dadurch gekennzeichnet, dass** das Wachs aus den bei Umgebungstemperatur festen und starren Wachsen mit einem Schmelzpunkt höher als oder gleich 30°C, insbesondere höher als oder gleich 45°C, vor allem höher als oder gleich 55°C ausgewählt ist.

**12.** Zusammensetzung nach einem der vorhergehenden Ansprüche, **dadurch gekennzeichnet, dass** das Wachs ausgewählt ist aus den Kohlenwasserstoffwachsen, wie Bienenwachs, Lanolinwachs, Chinawachsen, Sumac-Wachs, Paraffinen, Polyethylenwachsen, Wachscopolymeren sowie ihren Estern; Wachsen, die erhalten wurden durch katalytische Hydrogenierung von tierischen oder pflanzlichen Ölen mit linearen oder verzweigten $C_8$-$C_{32}$-Fettketten, wie partiell hydrogeniertes transisometrisiertes Jojobaöl, hydrogeniertes Sonnenblumenöl, hydrogeniertes Rizinusöl, hydrogeniertes Kopraöl, hydrogeniertes Lanolinöl und di(Drimethylol-1,1,1-propan)tetrastearat, und Wachsen, die durch Hydrogenieren von mit Cetylalkohol verestertem Rizinusöl erhalten werden.

**13.** Zusammensetzung nach einem der Ansprüche 1 bis 11, **dadurch gekennzeichnet, dass** das Wachs ausgewählt ist aus Wachsen mit einem Klebevermögen von mehr als oder gleich 0,7 N.s, insbesondere mehr als oder gleich 1 N.s, und einer Härte von weniger als oder gleich 3,5 MPa.

**14.** Zusammensetzung nach dem vorhergehenden Anspruch, **dadurch gekennzeichnet, dass** das Wachs aus den $C_{20}$-$C_{40}$-Alkyl(hydroxystearyloxy)stearaten ausgewählt ist.

**15.** Zusammensetzung nach einem der Ansprüche 1 bis 12, **dadurch gekennzeichnet, dass** das Wachs aus den Wachsen mit einem Anfangsschmelzpunkt höher als oder gleich 45°C, vor allem höher als oder gleich 50°C, insbesondere höher als oder gleich 55°C und ganz besonders höher als oder gleich 60°C ausgewählt ist.

**16.** Zusammensetzung nach dem vorhergehenden Anspruch 1, **dadurch gekennzeichnet, dass** das Wachs ausgewählt ist aus Carnaubawachs, Reiskleiewachs, Candellilawachs, Ouricurrywachs, Montanwachs, Ozokerit, den durch die Fisher-Tropsch-Synthese erhaltenen Wachsen, hydrogeniertem Jojobaöl, di-(Trimethylol-1,1,1-propan) tetra-behenat, den durch katalytische Hydrogenierung von mit Stearinalkohol verestertem Olivenöl erhaltenen Wachsen, den mikrokristallinen Wachsen und den Polyethylenwachsen.

**17.** Zusammensetzung nach einem der vorhergehenden Ansprüche, **dadurch gekennzeichnet, dass** der Gesamtgehalt an Wachs 25 bis 70 %, vor allem 25 bis 65 % und insbesoandere 25 bis 60 % oder sogar 25 bis 55 Gew.-%, bezogen auf das Gesamtgewicht der Zusammensetzung, beträgt.

**18.** Zusammensetzung nach einem der vorhergehenden Ansprüche, **dadurch gekennzeichnet, dass** das in dem nicht wässrigen Lösungsmittelmedium lösliche Polymer mit mindestens einem kristallisierbaren Teil eine molare Masse von 200 bis 1 000 000 g/mol, insbesondere von 500 bis 500 000 g/mol und ganz besonders von 1000 bis 300 000 g/mol besitzt.

**19.** Zusammensetzung nach einem der vorhergehenden Ansprüche, **dadurch gekennzeichnet, dass** der kristallisierbare Teil des in dem nicht wässrigen Lösungsmittelmedium löslichen Polymers mit mindestens einem kristallisierbarem Teil mindestens 5 %, insbesondere mindestens 10 % und höchstens 50 % und ganz besonders 30 bis 50 Gew.-% vom Gesamtgewicht des Polymers darstellt.

**20.** Zusammensetzung nach einem der vorhergehenden Ansprüche, **dadurch gekennzeichnet, dass** das in dem nicht wässrigen Lösungsmittelmedium lösliche Polymer mit mindestens einem kristallisierbaren Teil ausgewählt ist aus den Copolymeren von linearen und gesättigten $C_{12}$-$C_{3o}$-Alkyl(meth)acrylaten und linearen $C_4$-$C_{10}$- oder verzweigten oder cyclischen und/oder ungesättigten $C_4$-$C_{30}$-Alkyl(meth)acrylaten, den Copolymeren von Vinylestern mit linearen und gesättigten $C_{12}$-$C_{30}$-Alkylgruppen und Vinylestern mit linearen $C_4$-$C_{10}$- oder verzweigten oder cyclischen und/oder ungesättigten $C_4$-$C_{30}$-Alkylgruppen, den Polykondensaten vom Typ Polyamid, das sich aus der Kondensation zwischen ($\alpha$) mindestens einer Säure, die aus den mindestens 32 Kohlenstoffatomen umfassenden Dicarbonsäuren ausgewählt ist, und ($\beta$) einem Diaminalkylen ergibt, wobei das Polykondensat mindestens eine Endcarbonsäuregruppe umfasst, die mit mindestens einem Monoalkohol oder einem Monoamin verestert oder amidiert ist, umfassend 12 bis 30 lineare und gesättigte Kohlenstoffatome, und den lipophilen Polyesterpolykondensaten, deren Enden mit einer Säure oder einem kristallisierbaren Alkohol verestert sind, der aus einer linearen gesättigten $C_{12}$-$C_{30}$-Kohlenstoffkette besteht.

**21.** Zusammensetzung nach einem der vorhergehenden Ansprüche, dass das in dem nicht wässrigen Lösungsmittelmedium lösliche Polymer mit mindestens einem kristallisierbaren Teil ausgewählt ist aus den Copolymeren Vinylacetawinylstearat, Vinylacetat/Allylstearat, Vinylacetat/Ethylen, Ethylendiamin/Stearyldilinoleat, den sequenzierten Copolymeren, hydrogeniertes Butadien/Isopren und 12-Polyhydroxystearinsäure, die an einen ihrer Enden mit Stearinsäure verestert ist.

**22.** Zusammensetzung nach einem der vorhergehenden Ansprüche, **dadurch gekennzeichnet, dass** das in dem nicht wässrigen Lösungsmittelmedium lösliche Polymer mit mindestens einem kristallisierbaren Teil in einem Gehalt von 0,01 bis 30 %, vor allem 0,1 bis 20 % und insbesondere 1 bis 10 Gew.-%, bezogen auf das Gesamtgewicht der Zusammensetzung, vorliegt.

**23.** Zusammensetzung nach einem der vorhergehenden Ansprüche, **dadurch gekennzeichnet, dass** sie außerdem mindestens ein filmbildendes Polymer umfasst.

**24.** Zusammensetzung nach einem der vorhergehenden Ansprüche, **dadurch gekennzeichnet, dass** sie außerdem mindestens einen Farbstoff umfasst.

**25.** Zusammensetzung nach einem der vorhergehenden Ansprüche, **dadurch gekennzeichnet, dass** sie außerdem

mindestens einen Füllstoff umfasst.

26. Zusammensetzung nach einem der vorhergehenden Ansprüche, **dadurch gekennzeichnet, dass** sie außerdem mindestens einen kosmetisch annehmbaren Zusatz umfasst, ausgewählt aus den Antioxidantien, Konservierungsstoffen, Duftstoffen, Neutralisierungsmitteln, Weichmachern, Fasern, Geliermitteln, kosmetischen Wirkstoffen und ihren Mischungen.

27. Zusammensetzung nach einem der vorhergehenden Ansprüche, **dadurch gekennzeichnet, dass** sie eine Fließschwelle $\tau_c$, gemessen durch Schwingungsrheologie ($\gamma$ = 1 Hz), von 10 bis 200 Pa und insbesondere von 20 bis 100 Pa besitzt.

28. Verfahren zur Herstellung einer Zusammensetzung, wie sie in einem der Ansprüche 1 bis 27 definiert ist, **dadurch gekennzeichnet, dass** es mindestens einen Schritt der Dispersion mindestens eines Wachses im Zustand von Teilchen mit einer Abmessung von 0,5 bis 30 µm in mindestens einem nicht wässrigen Lösungsmittel umfasst, wobei das Lösungsmittel auf einer Temperatur ist, die niedriger als die Schmelztemperatur des Wachses im Zustand von Teilchen ist.

29. Verfahren nach dem vorhergehenden Anspruch, **dadurch gekennzeichnet, dass** die Dispersion bei Umgebungstemperatur durchgeführt wird.

30. Verfahren nach Anspruch 28 oder 29, **dadurch gekennzeichnet, dass** die Abmessung 1 bis 20 µm und insbesondere 5 bis 10 µm beträgt.

31. Verfahren nach einem der Ansprüche 28 bis 30, **dadurch gekennzeichnet, dass** das Wachs ausgewählt ist aus Carnaubawachs, Kunstwachs, den Wachsen, die aus einer Mischung von Carnaubawachs und Polyethylenwachs bestehen, den Wachsen, die aus einer Mischung von Carnaubawachs und Kunstwachs bestehen, den Polyethylenwachsen und den Polytetrafluorethylenwachsen.

32. Verfahren nach einem der Ansprüche 28 bis 31, **dadurch gekennzeichnet, dass** man zuvor mindestens ein Wachs, wie es in einem der Ansprüche 11 bis 16 definiert ist, in geschmolzener Form in das Lösungsmittel einführt und man die auf diese Weise erhaltene Mischung unter Rühren oder Kneten bis zu einer Temperatur abkühlen lässt, die mindestens niedriger als die Schmelztemperatur des Wachses im Zustand von Teilchen ist.

33. Verfahren nach einem der Ansprüche 28 bis 32, **dadurch gekennzeichnet, dass** das nicht wässrige Lösungsmittel so ist, wie es in einem der Ansprüche 1 oder 5 bis 7 definiert ist.

34. Verfahren zum Schminken von Keratinfasern, **dadurch gekennzeichnet, dass** man auf die Keratinfasern, insbesondere die Wimpern, eine Zusammensetzung aufträgt, wie sie in einem der Ansprüche 1 bis 27 definiert ist oder wie sie durch ein Verfahren, wie es in einem der Ansprüche 28 bis 33 definiert ist, erhalten wird.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 5480632 A
- EP 847752 A
- FR 2792190 A
- FR 2232303 A
- EP 6921217 A
- EP 686858 A
- US 5472798 A
- US 3802841 A
- FR 2079785 A
- EP 0360728 A1
- EP 0549494 A
- FR 2701198
- FR 2605505
- EP 792603 A
- EP 663161 A


### Littérature non-brevet citée dans la description


- **G. Couarraze ; J.L. Grossiord.** Initiation à la rhéologie. Edition Lavoisier-Tec, 1991
- **B. Boutevin et al.** Study of morphological and mechanical properties of PP/PBT. *Polymer Bulletin,* 1995, vol. 34, 117-123
- **P. Rangarajan et al.** Morphology of semi-crystalline block copolymers of ethylene-(ethylene-alt-propylene). *Macromolecules,* 1993, vol. 26, 4640-4645
- **P. Richter et al.** Polymer agregates with crystalline cores : the system poly(ethylene)-poly(ethylene-propylene). *Macromolécules,* 1997, vol. 30, 053-1068
- **I.W. Hamley.** Cristallization in block copolymers. *Advances in Polymer Science,* 1999, vol. 148, 113-137
- **R. PIGEON ; P. ALLARD.** *Chimie Macromoléculaire Appliquée,* 1974, vol. 40/41 (600), 139-158